(19) Europäisches Patentamt
European Patent Office
Office européen des brevets

(11) EP 2 222 272 B1

(12) **EUROPEAN PATENT SPECIFICATION**

(45) Date of publication and mention of the grant of the patent:
**07.11.2012 Bulletin 2012/45**

(21) Application number: **09784868.3**

(22) Date of filing: **05.08.2009**

(51) Int Cl.:
*A61K 9/00* (2006.01) *A61K 31/427* (2006.01)
*A61P 11/00* (2006.01) *C07D 413/12* (2006.01)
*C07D 417/12* (2006.01) *C07D 277/00* (2006.01)

(86) International application number:
**PCT/GB2009/001920**

(87) International publication number:
**WO 2010/015818 (11.02.2010 Gazette 2010/06)**

(54) **Respiratory disease treatment**

Behandlung von Atemwegserkrankungen

Traitement de maladie respiratoires

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **07.08.2008 GB 0814488**
**24.12.2008 GB 0823568**

(43) Date of publication of application:
**01.09.2010 Bulletin 2010/35**

(73) Proprietor: **Pulmagen Therapeutics (Inflammation) Limited**
**Burnham**
**Slough**
**Buckinghamshire SL1 8DF (GB)**

(72) Inventors:
- **FINCH, Harry**
  **Essex CM19 5TR (GB)**
- **FOX, Craig**
  **Essex CM19 5TR (GB)**
- **SAJAD, Mohammed**
  **Essex CM19 5TR (GB)**

(74) Representative: **Simcox, Michael Thomas et al**
**D Young & Co LLP**
**120 Holborn**
**London EC1N 2DY (GB)**

(56) References cited:
**WO-A1-02/13812 US-A- 6 127 394**

- **CAZZOLA ET AL: "Treating systemic effects of COPD" TRENDS IN PHARMACOLOGICAL SCIENCES, ELSEVIER, HAYWARTH, GB, vol. 28, no. 10, 2 October 2007 (2007-10-02), pages 544-550, XP022282058 ISSN: 0165-6147**
- **MUELLER C ET AL: "Peroxisome proliferator-activated receptor gamma ligands attenuate immunological symptoms of experimental allergic asthma" ARCHIVES OF BIOCHEMISTRY AND BIOPHYSICS, ACADEMIC PRESS, US, vol. 418, no. 2, 15 October 2003 (2003-10-15), pages 186-196, XP004458908 ISSN: 0003-9861**
- **NARALA VENKATA R ET AL: "Pioglitazone is as effective as dexamethasone in a cockroach allergen-induced murine model of asthma" RESPIRATORY RESEARCH, BIOMED CENTRAL LTD., LONDON, GB, vol. 8, no. 1, 4 December 2007 (2007-12-04), page 90, XP021031353 ISSN: 1465-9921**
- **DATABASE WPI Week 20085 Thomson Scientific, London, GB; AN 2008-A77102 XP002553092 MATSUI Y, OGATA T,OHSUMI J, WADA K: "Optically active thiazlidine derivative activating peroxisome proliferation factor receptor gamma for treating diabetes, hyperglycemia, is prepared by optically resolving racemic mixture of thiazolidine monohydrochloride" & WO 2007/100027 A1 (SANKYO CO LTD) 7 September 2007 (2007-09-07) & WO 2007/100027 A1 (DAIICHI SANKYO CO LTD [JP]; OHSUMI JUN; WADA KUNIO; MATSUI YUMI; OGATA) 7 September 2007 (2007-09-07)**

EP 2 222 272 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

This invention relates to the use of the substantially pure 5R enantiomers of the known glitazone drug class, such as the known pharmaceutical products pioglitazone and rosiglitazone, for pulmonary administration by inhalation, for treatment of inflammatory respiratory diseases.

Background to the invention

A broad spectrum of respiratory diseases and disorders has been recognized, many of which have overlapping and interacting etiologies. Two of the most widespread and prevalent of these diseases are chronic obstructive pulmonary disease (COPD) and asthma. Respiratory diseases have a significant inflammatory component. For example, current therapy for COPD and severe asthma focuses mainly on the reduction of symptoms using short and long acting bronchodilators either as monotherapies or combinations of long acting $\beta_2$ agonist bronchodilators with inhaled corticosteroids (ICS). The disappointing anti-inflammatory data for ICS either alone or in combination with β2 agonists has intensified the search for an effective anti-inflammatory drug for COPD. COPD is clearly a chronic inflammatory disorder that involves complex interactions between cells of the innate and acquired immune response both in the lung and potentially also systemically. One hypothesis under intense investigation is whether novel, demonstrably anti-inflammatory agents can halt or slow the functional decline characteristic of COPD. Reducing the frequency and severity of exacerbations has become an increasingly important target for COPD therapy as the prognosis for patients following exacerbations is poor. Anti-inflammatory therapy in COPD, and in asthma, is expected to reduce the frequency and severity of exacerbations. It is also desirable that decline in lung function and quality of life are also ameliorated with treatment.

Hence, new treatments for inflammatory respiratory diseases, including asthma, COPD, allergic airway syndrome, bronchitis, cystic fibrosis, emphysema and pulmonary fibrosis (including idiopathic pulmonary fibrosis), are constantly sought.

Peroxisome Proliferation Receptor gamma receptor (PPARγ) agonists are a class of drugs which increase sensitivity to glucose in diabetic patients. Physiological activation of PPARγ is believed to increase the sensitivity of peripheral tissues to insulin, thus facilitating the clearance of glucose from the blood and producing the desired antidiabetic effect.

Many PPARγ agonists are known from the patent and other literature, but currently only two are approved for clinical use in diabetes; Rosiglitazone and Pioglitazone. See Campbell IW, Curr Mol Med. 2005 May; 5(3):349-63. Both of these compounds are thiazolidinediones ("TZDs" or "glitazones"), and are in practice administered by the oral route for systemic delivery.

In addition to its effect on glucose metabolism, a variety of reports have been published which demonstrate that rosiglitazone also exerts anti-inflammatory effects. For instance, (i) rosiglitazone has been reported to exert effects in diabetic patients consistent with an anti-inflammatory effect (Haffner et al., Circulation. 2002 Aug 6;106(6):679-84, Marx et al., Arterioscler. Thromb. Vasc. Biol. 2003 Feb 1;23(2):283-8); (ii) Rosiglitazone has been reported to exert anti-inflammatory effects in a range of animal models of inflammation, including: carageenan-induced paw oedema (Cuzzocrea et al., Eur. J. Pharmacol. 2004 Jan 1;483(1):79-93), TNBS-induced colitis (Desreumanux et al., J. Exp. Med. 2001 Apr 2;193(7):827-38, Sanchez-Hidalgo et al., Biochem. Pharmacol. 2005 Jun 15;69(12):1733-44), experimental encephalomyelitis (Feinstein et al., Ann. Neurol. 2002 Jun;51 (6):694-702) collagen-induced (Cuzzocrea et al., Arthritis Rheum. 2003 Dec;48(12):3544-56) and adjuvant-induced arthritis (Shiojiri et al., Eur. J. Pharmacol. 2002 Jul 19;448(2-3):231-8), carageenan-induced pleurisy (Cuzzocrea et al., Eur. J. Pharmacol. 2004 Jan 1;483(1):79-93), ovalbumin-induced lung inflammation (Lee et al., FASEB J. 2005 Jun;19(8):1033-5) and LPS-induced lung tissue neutrophilia (Birrell et al., Eur. Respir. J. 2004 Jul;24(1):18-23) and (iii) rosiglitazone has been reported to exert anti-inflammatory effects in isolated cells, including iNOS expression in murine macrophages (Reddy et al., Am. J. Physiol. Lung Cell. Mol. Physiol. 2004 Mar;286(3):L613-9), TNFα-induced MMP-9 activity in human bronchial epithelial cells (Hetzel et al., Thorax. 2003 Sep; 58(9):778-83), human airway smooth muscle cell proliferation (Ward et a/., Br. J. Pharmacol. 2004 Feb; 141(3):517-25) and MMP-9 release by neutrophils (WO 0062766 - is this for year 2000?). PPARγ agonists have also been shown to be effective in models of pulmonary fibrosis (Milam et al., Am. J. Physiol. Lung Cell. Mol. Physiol, 2008, 294(5):L891-901) and pulmonary arterial hypertension (Crossno et al., Am. J. Physiol. Lung Cell. Mol. Physiol, 2007, 292(4):L885-897).

Based on observations of anti-inflammatory activity in cells relevant to the lung, the utility of other PPARγ agonists has been suggested for the treatment of inflammatory respiratory disorders including asthma, COPD, cystic fibrosis and pulmonary fibrosis. See WO0053601, WO0213812 and WO0062766. These suggestions include administration by both the systemic oral and pulmonary inhalation routes.

Unfortunately, PPARγ agonists also have unwanted cardiovascular effects, including haemodilution, peripheral and pulmonary oedema and congestive heart failure (CHF). These effects are also believed to result from activation of PPARγ. In particular, a significant effort has been devoted to investigating the hypothesis that PPARγ agonists disturb the normal maintenance of fluid balance via binding to the PPARγ receptor in the kidney. See Guan et al, Nat. Med.

2005;11(8):861-6 and Zhang et. al., Pro.c Natl. Acad. Sci. USA. 2005 28; 102(26):9406-11. Treatment with PPARγ agonists by the oral route for systemic delivery is also associated with an unwanted increase in body weight.

COPD patients are known to be at a higher risk than other clinical populations from congestive heart failure (CHF) (Curkendall et al, Ann Epidemiol, 2006;16: 63-70, Padeletti M et al, Int J Cardiol. 2008;125(2):209-15) and so it is important that systemic activation of the PPARγ receptors is kept to a minimum in these patients to avoid increasing the likelihood of CHF being observed. Administering respiratory drugs by the inhaled route is one approach to target the lung with an anti-inflammatory agent whilst keeping systemic exposure of the drug low, thus reducing the likelihood of systemic activity and observation of side effects.

Pioglitazone has structural formula (I)

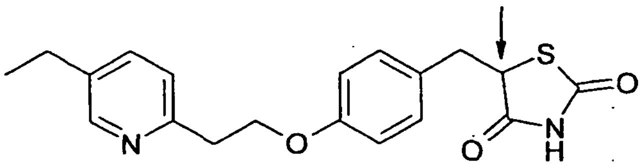

(I)

and can be named as 5-{4-[2-(5-ethylpyridin-2-yl)ethoxy]benzyl}-1,3-thiazolidine-2,4-dione. The carbon atom in the 5-position of the thiazolidine-dione ring of pioglitazone, indicated by an arrow in formula (I) above, is asymmetric, so pioglitazone has two enantiomers, the 5R and 5S enantiomers.

Rosiglitazone has the structural formula (II) and can be named as 5-(4-{2-[methyl (pyridin-2-yl)amino]ethoxylbenzyl}-1,3-thiazolidine-2,4-dione. The carbon atom in the 5-position of the thiazolidine-dione ring of rosiglitazone, indicated by an arrow in formula (II) below, is also asymmetric, so rosiglitazone also has two enantiomers, the 5R and 5S enantiomers.

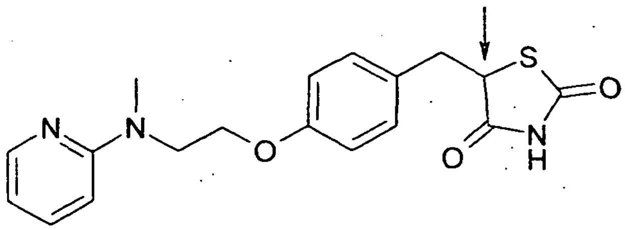

(II)

The 5S enantiomer of rosiglitazone has a higher binding affinity for the PPARγ receptor than the 5R enantiomer (30nM vs 2μM, Parks et al., 1998, Bioorg. Med. Chem. Lett. 8(24):3657-8). For another member of the glitazone class, Rivoglitazone, the 5S enantiomer also has higher receptor binding affinity than the 5R enantiomer (see page 13 of WO2007100027).

In practice, pioglitazone and rosiglitazone are administered for treatment of diabetes as a mixture of 5R and 5S enantiomers (a 1:1 racemic mixture) by the oral route for systemic delivery. The individual enantiomers of these compounds, and members of the glitazone family generally, are known to equilibrate rapidly *in vivo* after oral administration (see for example J.Clin. Pharmacol. 2007, 47, 323-33; Rapid Commun. Mass Spectrom. 2005, 19, 1125-9; J. Chromatography, 835 (2006), 40-46; Biopharmaceutics and Drug Disposition 1997, 18 (4), 305-24; Chem. Pharm. Bull 1984, 32, (11) 4460-65; T. J. Med. Chem. 1991, 34, 319-25) so there is no difference in practice between oral administration of either substantially pure isomer and oral administration of the racemic mixture. Specifically in relation to pioglitazone, it has been stated in a submission to the Federal Drug Administration (FDA) that there was no difference in activity following oral administration either of the racemate or the individual enantiomers in a rodent diabetes model (www.fda.gov/medwatch/SAFETY/2007/Sep PI/Actoplus Met PI.pdf):

*"(Pioglitazone) contains one asymmetric carbon, and the compound is synthesized and used as the racemic mixture. The two enantiomers of pioglitazone interconvert in vivo. No differences were found in the pharmacologic activity between the two enantiomers".*

The effects of pulmonary inhalation of rosiglitazone or pioglitazone (or indeed any other glitazone) in either racemic or single enantiomer form do not appear to have been studied. It appears that nothing has been published concerning the potential equilibration of the 5R and 5S enantiomers of either compound, or any other glitazone, when contacted directly with lung tissue.

The glitazone class of PPARγ agonists as a whole is characterised by the presence in the molecule of a thiazolidin-2,4-dione radical (A), often as part of a (thiazolidin-2,4,dione-5-yl)methylphenyl radical (B):

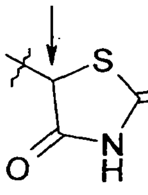
(A)

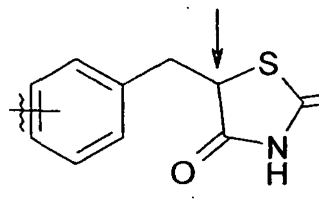
(B)

and the ring carbon atom indicated by the arrow is numbered as the 5-position of the thiazolidinone ring. The term "glitazone" as used herein refers to a PPARγ agonist compound whose structure includes a thiazolidin-2,4-dione radical (A), or a (thiazolidin-2,4,dione-5-yl)methylphenyl radical (B):

Besides the approved and marketed rosiglitazone and pioglitazone, there is a multitude of glitazones known from the patent and scientific literature. Known examples include the following:

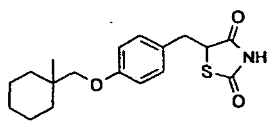
Ciglitazone

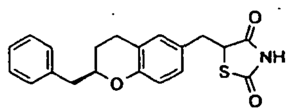
Englitazone

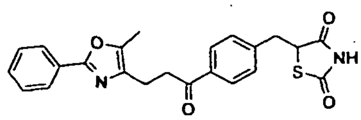
Darglitazone

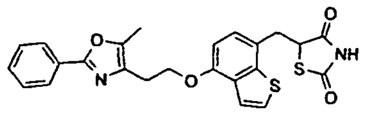
Edaglitazone

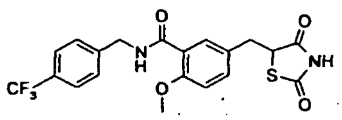
MK-0767

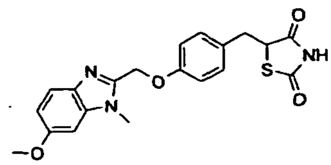
Rivoglitazone

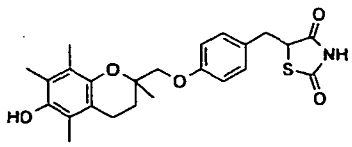
Troglitazone

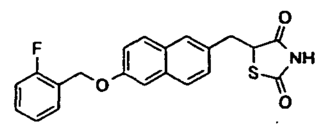
Netoglitazone

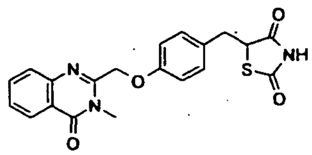
Balaglitazone

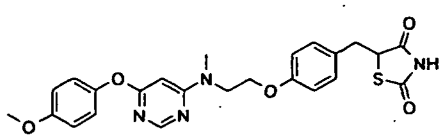
Lobeglitazone

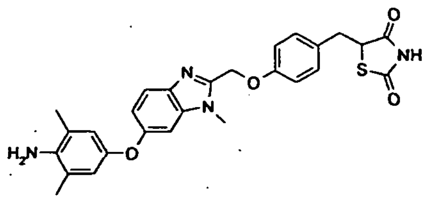
Inolitazone

Brief summary of the invention

This invention is based on the finding that, for treatment of inflammatory respiratory disease by inhalation, the 5R configuration of a glitazone is more effective than the 5S configuration. Proof of principle drives from an animal model of treatment of inflammatory respiratory disease by inhalation, in which the 5R-enantiomers of pioglitazone and rosiglitazone have been shown to be active, whereas the 5S enantiomers were essentially inactive. This finding leads to the conclusion that inhaled pulmonary administration of the 5R configuration of a glitazone, in particular the 5R-enantiomer of pioglitazone or rosiglitazone, allows the anti-inflammatory effect of the compound to be achieved more efficiently than by similar administration of the racemate, with all the concomitant reduced side effect benefits of lower systemic exposure than oral administration.

Brief description of the figures

Figure. 1 is a bar graph that illustrates the effect of intranasal administration to laboratory mice with vehicle (0.2% tween 80 in saline), 5S-pioglitazone (1 or 3 μg/kg), 5R-pioglitazone (1 or 3 μg/kg), or racemic pioglitazone (3 μg/kg) on the number of BAL cells induced by tobacco smoke 24 hours post the final exposure.

Figure. 2 is a bar graph that illustrates the effect of intranasal administration to laboratory mice with vehicle (0.2% tween 80 in saline), 5S-pioglitazone (1 or 3 μg/kg), 5R-pioglitazone (1 or 3 μg/kg), or racemic pioglitazone (3 μg/kg) on the number of BAL neutrophils induced by tobacco smoke 24 hours post the final exposure.

Figure. 3 is a bar graph that illustrates the effect of intranasal administration to laboratory mice with vehicle (0.2% tween 80 in saline), 5S-Rosiglitazone. (3 or 10 μg/kg), 5R-Rosiglitazone (3 or 10 μg/kg), or racemic Rosiglitazone (10 μg/kg) on the number of BAL cells induced by tobacco smoke 24 hours post the final exposure.

Figure. 4 is a bar graph that illustrates the effect of intranasal administration to laboratory mice with vehicle (0.2% tween 80 in saline), 5S-Rosiglitazone (3 or 10 μg/kg), 5R-Rosiglitazone (3 or 10 μg/kg), or racemic Rosiglitazone (10 μg/kg) on the number of BAL neutrophils induced by tobacco smoke 24 hours post the final exposure.

Detailed description of the invention

As used herein, the term "glitazone" has the meaning ascribed to it above, i.e., a PPARγ agonist compound whose structure includes a thiazolidin-2,4-dione radical (A), or a (thiazolidin-2,4,dione-5-yl)methylphenyl radical (B):

As used herein the term "pioglitazone" or "pioglitazone component" means the compound 5-{4-[2-(5-ethylpyridin-2-yl)ethoxy]benzyl}-1,3-thiazolidine-2,4-dione of formula (I) above, or a pharmaceutically acceptable salt thereof.

As used herein the term "rosiglitazone" or "rosiglitazone component" means the compound 5-(4-{2-[methyl (pyridin-2-yl)amino]ethoxy]benzyl}-1,3-thiazolidine-2,4-dione of formula (II) above, or a pharmaceutically acceptable salt thereof.

As used herein, the term "enantiomeric excess" or its abbreviation "e.e." is defined as the percentage:

$$((R-S)/(R+S)) \times 100 \text{ percent}$$

where R and S are the respective weight fractions of the R and S enantiomers in a sample. Thus for a glitazone sample containing 95% by weight of the 5R enantiomer and 5% of the 5S enantiomer, the enantiomeric excess of R over S enantiomer is ((95-5)/95+5)) x 100 = 90%.

In one aspect, the present invention provides a pharmaceutical composition adapted for pulmonary administration by inhalation, which composition comprises a glitazone, particularly pioglitazone or rosiglitazone, and one or more pharmaceutically acceptable carriers and/or excipients, wherein the glitazone content of the composition consists of at least 95% by weight of the 5R configuration and less than 5% of the 5S configuration.

In another aspect, the invention provides a glitazone, for example pioglitazone or rosiglitazone, for use in the treatment of inflammatory respiratory disease by pulmonary administration by inhalation, wherein the glitazone inhaled consists of at least 95% by weight of the 5R configuration and less than 5% of the 5S configuration.

In another aspect, the invention provides the use of a glitazone, for example pioglitazone or rosiglitazone, in the preparation of a medicament for the treatment of inflammatory respiratory disease by pulmonary administration by inhalation, wherein the glitazone content of the medicament consists of at least 95% by weight of the 5R configuration and less than 5% of the 5S configuration.

There is also described a use of a therapeutically effective amount of a glitazone, for example pioglitazone or rosiglitazone for the treatment of inflammatory respiratory disease comprising pulmonary administration to a subject suffering such disease by inhalation, wherein the glitazone inhaled consists of at least 95% by weight of the 5R configuration and less than 5% of the 5S configuration.

In all aspects of the invention, the glitazone component, for example the pioglitazone or rosiglitazone component, may be inhaled via the nose or the mouth. Preferably it is inhaled via the mouth.

In all aspects of the invention, the glitazone component, for example pioglitazone or rosiglitazone, should preferably contain as little of the 5S configuration as possible. For example, the 5R configuration may constitute at least 97%, or at least 98%, or at least 99% by weight of the glitazone component

In all aspects of the invention, the glitazone component, for example the pioglitazone or rosiglitazone component, may be accompanied by, or administered sequentially or concurrently with, one or more other therapeutic agents useful for the purpose of preventing and treating respiratory disorders, other than a PPARγ agonist.

In all aspects of the invention, currently the most preferred glitazone component is pioglitazone.

In all aspects of the invention, the wherein the inflammatory respiratory disease may be selected from, for example, mild asthma, moderate asthma, severe asthma, steroid resistant asthma, bronchitis, chronic obstructive pulmonary disease (COPD), cystic fibrosis, pulmonary edema, pulmonary embolism, pneumonia, pulmonary sarcoisosis, silicosis, pulmonary fibrosis, respiratory failure, acute respiratory distress syndrome, emphysema, chronic bronchitis, tuberculosis, and lung cancer.

The glitazone component can be in the form of a pharmaceutically acceptable salt. The term "pharmaceutically acceptable salt" refers to salts prepared from pharmaceutically acceptable inorganic and organic acids and bases.

Pharmaceutically acceptable inorganic bases include metallic ions. More preferred metallic ions include, but are not limited to, appropriate alkali metal salts, alkaline earth metal salts and other physiological acceptable metal ions. Salts derived from inorganic bases include aluminum, ammonium, calcium, copper, ferric, ferrous, lithium, magnesium, manganic salts, manganous, potassium, sodium, zinc, and the like and in their usual valences. Exemplary salts include aluminum, calcium, lithium, magnesium, potassium, sodium and zinc. Particularly preferred are the ammonium, calcium, magnesium, potassium, and sodium salts.

Salts derived from pharmaceutically acceptable organic non-toxic bases include salts of primary, secondary, and tertiary amines, including in part, trimethylamine, diethylamine, N,N'-dibenzylethylenediamine, chloroprocaine, choline, diethanolamine, ethylenediamine, meglumine (N-methylglucamine) and procaine; substituted amines including naturally occurring substituted amines; cyclic amines; and quaternary ammonium cations. Examples of such bases include arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Illustrative pharmaceutically acceptable acid addition salts of the compounds of the present invention can be prepared from the following acids, including, without limitation formic, acetic, propionic, benzoic, succinic, glycolic, gluconic, lactic, maleic, malic, tartaric, citric, nitric, ascorbic, glucuronic, maleic, fumaric, pyruvic, aspartic, glutamic, benzoic, hydrochloric, hydrobromic, hydroiodic, isocitric, xinafoic, tartaric, trifluoroacetic, pamoic, propionic, anthranilic, mesylic, napadisylate, oxalacetic, oleic, stearic, salicylic, p-hydroxybenzoic, nicotinic, phenylacetic, mandelic, embonic (pamoic), methanesulfonic, phosphoric, phosphonic, ethanesulfonic, benzenesulfonic, pantothenic, toluenesulfonic, 2-hydroxyethanesulfonic, sulfanilic, sulfuric, salicylic, cyclohexylaminosulfonic, algenic, β-hydroxybutyric, galactaric and galacturonic acids. Exemplary pharmaceutically acceptable salts include the salts of hydrochloric acid and hydrobromic acid.

Compositions of the invention are useful for treatment of inflammatory respiratory disorders, for example asthma (mild, moderate or severe), e.g., bronchial, allergic, intrinsic, extrinsic, exercise-induced, drug-induced (including aspirin and NSAID-induced) and dust-induced asthma, steroid resistant asthma, bronchitis including infectious and eosinophilic bronchitis, chronic obstructive pulmonary disease (COPD), cystic fibrosis, pulmonary fibrosis including cryptogenic fibrosing alveolitis, idiopathic pulmonary fibrosis, idiopathic interstitial prieumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension (including pulmonary arterial hypertension); antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus, pulmonary edema, pulmonary embolism, pneumonia, pulmonary sarcoidosis, silicosis, farmer's lung and related diseases; hypersensitivity pneumonitis, respiratory failure, acute respiratory distress syndrome, emphysema, chronic bronchitis, tuberculosis, and lung cancer. In particular, the uses and compositions of the present invention encompass the prevention and treatment of the respiratory disorder, COPD.

As used herein, the term "chronic obstructive pulmonary disease" or "COPD" refers to a set of physiological symptoms including chronic bronchitis, chronic cough, expectoration, exertional dyspnea and a significant, progressive reduction in airflow that may or may not be partly reversible. Emphysema may also be present in the lungs. COPD is a disease characterized by a progressive airflow limitation caused by an abnormal inflammatory reaction to the chronic inhalation of particles.

In subjects with the disorder, poor gas exchange in the lungs leads to decreased oxygen levels in the blood, increased levels of carbon dioxide and shortness of breath. Chronic airflow obstruction in COPD is complicated by the loss of lung elasticity resulting from enzymatic destruction of the lung parenchyma. Rather than a single pathologic condition, COPD is an umbrella term encompassing chronic obstructive bronchitis and emphysema.

Compositions suitable for administration by inhalation via the mouth or the nose are known, and may include

carriers and/or diluents that are known for use in such compositions. The composition may contain 0.01-99% by weight of the pioglitazone or rosiglitazone component. Preferably, a unit dose comprises the pioglitazone or rosiglitazone component in an amount of 1 μg to 50 mg.

The most suitable dosage level may be determined by any suitable method known to one skilled in the art. It will be understood, however, that the specific amount for any particular patient will depend upon a variety of factors, including the activity of the specific compound that is used, the age, body weight, diet; general health and.sex of the patient, time of administration, the route of administration, the rate of excretion, the use of any other drugs, and the severity of the disease undergoing treatment. Optimum dosages will be determined by clinical trial, as is required in the art.

Compositions of the invention may be used in combination with other therapeutic agents that are used in the treatment/prevention/suppression or amelioration of the diseases or conditions for which present compounds are useful. Such other therapeutic agents may be administered, by a route and in an amount commonly used therefore, contemporaneously or sequentially with the glitazone component, particularly the pioglitazone or rosiglitazone component. When a compound of the invention is used contemporaneously with one or more other therapeutic agents, a pharmaceutical composition containing such other therapeutic agents in addition to the pioglitazone component is preferred. Accordingly, the pharmaceutical compositions of the present invention include those that also contain one or more other active ingredients, in addition to the glitazone component, particularly the pioglitazone or rosiglitazone component.

Suitable therapeutic agents for a combination therapy with the glitazone compositions, particularly the pioglitazone or rosiglitazone compositions, of the invention include one or more other therapeutic agents selected from anti-inflammatory agents, bronchodilators, mucolytic agents, antitussive agents, leukotriene inhibitors, and antibiotics.

Suitable therapeutic agents for a combination therapy with the glitazone compositions, particularly the pioglitazone or rosiglitazone compositions, of the invention include: (1) a steroid drug such as a corticosteroid, for example beclomethasone, (e.g., as the mono or the dipropionate ester), flunisolide, fluticasone (e.g., as the propionate or furoate ester), ciclesonide, mometasone (e.g., as the furoate ester), mometasone desonide, rofleponide, hydrocortisone, prednisone, prednisolone, methyl prednisolone, naflocort, deflazacort, halopredone acetate, fluocinolone acetonide, fluocinonide, clocortolone, tipredane, prednicarbate, alclometasone dipropionate, halometasone, rimexolone, deprodone propionate, triamcinolone, betamethasone, fludrocortisone, desoxycorticosterone, etiprendnol dicloacetate and the like. Steroid drugs can additionally include steroids in clinical or pre-clinical development for respiratory diseases such as GW-685698, GW-799943, GSK 870086, QAE397, NCX-1010, NCX-1020, NO-dexamethasone, PL-2146, NS-126 (formerly ST-126) and compounds referred to in international patent applications W00212265, W00212266, W002100879, WO03062259, WO03048181 and WO03042229. Steroid drugs can also additionally include next generation molecules in development with reduced side effect profiles such as selective glucocorticoid receptor agonists (SEGRAs), including ZK-216348 and compounds referred to in international patent applications WO-00032585, WO-000210143, WO-2005034939, WO-2005003098, WO-2005035518 and WO-2005035502 and functional equivalents and functional derivatives thereof; (2) a β2-adrenoreceptor agonist, such as albuterol, bambuterol, terbutaline, fenoterol, formoterol, formoterol fumarate, salmeterol, salmeterol xinafoate, arformoterol, arfomoterol tartrate, indacaterol (QAB-149), carmoterol, picumeterol, BI 1744 CL, GSK159797, GSK59790, GSK159802, GSK642444, GSK678007, GSK96108, clenbuterol, procaterol, bitolterol, and broxaterol,TA-2005 and also compounds of EP1440966,-JP05025045, WO93/18007, WO99/64035, US2002/0055651, US2005/0133417, US2005/5159448, WO00/075114, WO01/42193, WO01/83462, WO02/66422, WO02/70490, WO02/76933, WO03/24439, WO03142160, WO03/42164, WO03/72539, WO03/91204, WO03/99764, WO04/16578, WO04/016601, WO04/22547, WO04/3292 WO04/33412, WO04/37768, WO04/37773 WO04/37807, W00439762, WO04/39766. WO04/45618, WO04/46083, WO04/71388, WO04/80964 EP1460064, WO04/087142, WO04/89892, EP01477167, US2004/0242622, US2004/0229904, WO04/108675, WO04/108676, WO05/033121, WO05/040103, WO05/044787, WO04/071388 WO05/058299, WO05/058867, WO05/065650, WO05/066140, WO05/070908, WO05/092840; WO05/092841, WO05/092860, WO05/092887, WO05/092861, WO05/090288, WO05/092087, WO05/080324, WO05/080313, US20050182091. US20050171147, WO05/092870, WO05/077361, DE10258695, WO05/111002, WO05/111005, WO05/110990, US2005/0272769 WO05/110359, WO05/121065, US2006/0019991, WO06/016245, WO06/014704, WO06/031556, WO06/032627, US2006/0106075, US2006/0106213, WO06/051373. WO06/056471 WO08/096112, WO08/104790, WO08/096119, WO08/096112; (3) a leukotriene modulator, for example, montelukast or pranlukast; (4) anticholinergic agents, for example, selective muscarinic-3 (M3) receptor antagonists such as ipratropium bromide, tiotropium, tiotropium bromide (Spiriva®), glycopyrollate, NVA237, LAS34273, GSK656398, GSK233705, GSK 573719, LAS35201, QAT370 and oxytropium bromide; (5) phosphodiesterase-IV (PDE-IV) inhibitors, for example, roflumilast or cilomilast; (6) an antitussive agent, such as codeine or dextramorphan; (7) a non-steroidal anti-inflammatory agent (NSAID), for example, ibuprofen or ketoprofen; (8) a mucolytic, for example, N acetyl cysteine or fudostein; (9) a expectorant/mucokinetic modulator, for example, ambroxol, hypertonic solutions (e.g., saline or mannitol) or surfactant; (10) a peptide mucolytic, for example, recombinant human deoxyribonoclease I (dornase-alfa and rhDNase) or helicidin; (11) antibiotics, for example, azithromycin, tobramycin and aztreonam; and (12) p38 MAP kinase inhibitors such as GSK 856553 and GSK 681323.

In one aspect, the invention provides for the use of inhaled administration of the glitazone compositions, particularly the pioglitazone or rosiglitazone compositions, of the invention in combination with other anti-inflammatory drugs and bronchodilator drug combinations (i.e. triple combination product), including but not limited to salmeterol xinafoate/fluticasone propionate (Advair/Seretide®), formoterol fumarate/budesonide (Symbicort®), formoterol fumarate/mometasone furoate, formoterol fumarate/beclometasone dipropionate (Foster®), formoterol fumarate/fluticasone propionate (FlutiForm®). Indacaterol/mometasone furoate, Indacaterol/QAE-397, GSK159797/GSK 685698, GSK159802/GSK 685698, GSK642444/GSK 685698, formoterol fumarate/ciciesonide, arformoterol tartrate/ciclesonide.

In another aspect, the invention provides for the use of inhaled administration of the glitazone compositions, particularly the pioglitazone or rosiglitazone compositions, of the invention in combination with other bronchodilator drug combinations, particularly B2 agonist/m3 antagonist combinations (i.e. triple combination product), including but not limited to salmeterol xinafoate/tiotropium bromide, formoterol fumarate/tiotropium bromide, BI 1744 CUtiotropium bromide, indacaterol/NVA237, indacterol/QAT-370, formoterol/ LAS34273, GSK159797/GSK 573719, GSK159802/GSK 573719, GSK642444/GSK 573719, GSK159797/GSK 233705, GSK159802/GSK 233705, GSK642444/GSK 233705, and compounds which possess both β2 agonist and M3 antagonist activity in the same molecule (dual functionality) such as GSK 961081.

Thus in another aspect, the invention provides a kit for treatment of respiratory disorders in a subject, the kit comprising one dosage form comprising a composition adapted for pulmonary administration by inhalation, which composition comprises a glitazone, particularly pioglitazone or rosiglitazone, and one or more pharmaceutically acceptable carriers and/or excipients, wherein the glitazone content of the composition consists of at least 95% by weight of the 5R enantiomer and less than 5% of the 5S enantiomer, and a second dosage form comprising another therapeutic agent, for example as discussed above, selected from anti-inflammatory agents, bronchodilators, mucolytic agents, antitussive agents, leukotriene inhibitors,and antibiotics.

For delivery by inhalation, the active compound is preferably in the form of microparticles. These may be prepared by a variety of techniques, including spray-drying, freeze-drying and micronisation. Following size reduction to produce microparticles, particle size distribution (PSD) of the compound is examined and generally described in the art by specifying d10, d50 and d90 values. The average particle size, i.e. the average equivalent diameter, is defined as the diameter where 50 mass-% (of the particles) of the powder have a larger equivalent diameter, and the other 50 mass-% have a smaller equivalent diameter. Hence the average particle size is denoted as equivalent d50. For inhaled use a d50 of less than 10 microns, preferably less than 5 microns is desired.

By way of example, a composition of the invention may be prepared as a suspension for delivery from a nebuliser or as an aerosol in a liquid propellant, for example for use in a pressurised metered dose inhaler (PMDI). Propellants suitable for use in a PMDI are known to the skilled person, and include CFC-12, HFA-134a, HFA-227, HCFC-22 ($CCl_2F_2$) and HFA-152 ($CH_4F_2$ and isobutane).

In a preferred embodiment of the invention, a composition of the invention is in dry powder form, for delivery using a dry powder inhaler (DPI). Many types of DPI are known.

Microparticles for delivery by inhalation may be formulated with excipients that aid delivery and release. For example, in a dry powder formulation, microparticles may be formulated with large carrier particles that aid flow from the DPI into the lung. Suitable carrier particles are known, and include lactose particles; they may have a mass median aerodynamic diameter of greater than 90 μm.

Aerosol generation can be carried out using, for example, pressure-driven jet atomizers or ultrasonic atomizers, preferably using propellant-driven metered aerosols or propellant-free administration of micronized active compounds from, for example, inhalation capsules or other "dry powder" delivery systems.

The compositions may be dosed as described depending on the inhaler system used. In addition to the active compounds, the administration forms may additionally contain excipients, such as, for example, propellants (e.g., Frigen in the case of metered aerosols), surface-active substances, emulsifiers, stabilizers, preservatives, flavourings, fillers (e.g. lactose in the case of powder inhalers) or, if appropriate, further active compounds.

For the purposes of inhalation, a large number of systems are available with which aerosols of optimum particle size can be generated and administered, using an inhalation technique which is appropriate for the patient. In addition to the use of adaptors (spacers, expanders) and pear-shaped containers (e.g. Nebulator®, Volumatic®), and automatic devices emitting a puffer spray (Autohaler®), for metered aerosols, in particular in the case of powder inhalers, a number of technical solutions are available (e.g. Diskhaler®, Rotadisk®, Turbohaler® or the inhalers for example as described in EP-A-0505321).

<u>Methods of preparation of Glitazone Enantiomers</u>

Glitazones can be separated using chiral HPLC (see for example Methods 1-3 and 5-7 below). Chiral columns include CHIRALPAK AD, AD-H, AS-V, 50801, IA, IC, OD, OF, OG, OJ, OK, and OZ. Preferred chiral columns for HPLC are CHIRALPAK AD-H and CHIRALPAK IA using elution with ethanol and varying portions of TFA, preferably 0.05-0.2% TFA.

An alternative method of resolution, using pioglitazone as an example, is shown in Scheme 2 (see also Example 10).

Scheme 2

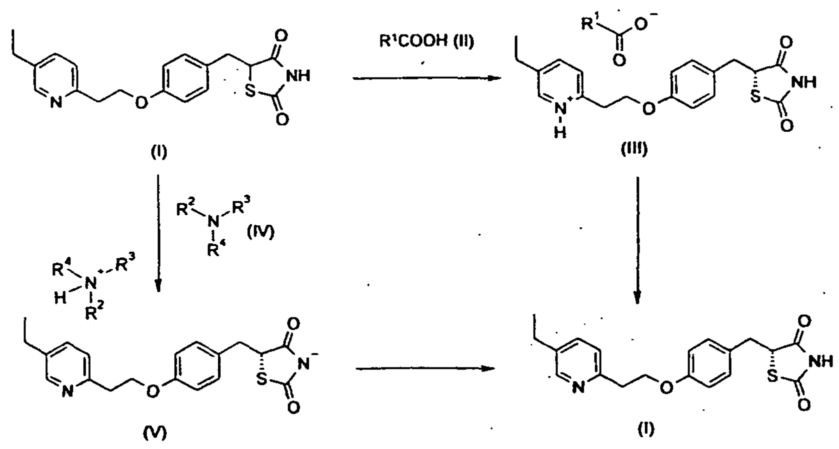


Recent review articles on optical resolution methods: E. Fogassy et. al., Tetrahedron: asymmetry, 2008, 19, 519-536; S.H.Wilen, Topics in Stereochemistry, Wiley-Interscience: NY, 1972, 6, 107 Eds. E.L. Eliel, N.L. Allingen P.Newman, Optical resolution Procedures of Chiral Compounds 1-3, Resolution Information Center, NY, 1978-1984; J.Jaques, S.H.Wilen, A. Collett, Enantiomers Racemates and Resolutions, Wiley-Interscience: NY, 1991; R.A. Scheldon, Chirotechnology, Marcel Dekker, NY, 1993; Optical Resolutions via diastereomeric salt formation, CRC Press, 2002, Ed. David Kozma.

Commonly used chiral acid resolving agents include, without limitation, dibenzoyl tartaric acid, dianisoyl tartaric acid, ditoluyl tartaric acid, tartaric acid, phencyphos, chlocyphos, anicyphos, 1,1'-binaphthyl-2,2'-diyl hydrogen phosphate, camphorsulfonic acid, bromo camphorsulfonic acid, camphoric acid, phenylethylsulfonic acid, malic acid, mandelic acid, 4-bromomandelic acid, 4-methylmandelic acid, lactic acid and chalcone sulfonic acids.

A more preferred set of chiral acid resolving agents include (-)-O,O'-dibenzoyl-L-tartaric acid (anhydrous), (-)-O, O'-dibenzoyl-L-tartaric acid monohydrate, (-)-di-O-p-toluoyl-L-tartaric acid, L-(+)-tartaric acid and (-)-di-p-anisolyl-L-tartaric acid.

The most preferred chiral acid resolving agent is (-)-O,O'-dibenzoyl-L-tartaric acid (anhydrous).

Chiral acid resolving agents can be used in different stoichiometries to effect resolutions. The above resolving agents can be used in a ratio of 1 part (I) to 10 parts of chiral acids (II), more preferably in a ratio of 1 part (I) to 5 parts of chiral acids (II), and most preferably in a ratio of 1 part (I) to 2 part of chiral acids (II). Even more preferred is a ratio of 1 part (I) to 1 part of chiral acids (II).

A variety of solvents may be useful to form diastereomeric salts (III) with chiral acids (II). Preferred solvents will include ethyl acetate, dichloromethane, tetrahydrofuran, 1,4-dioxane, acetone, acetonitrile, MeOH, EtOH, IPA, 1-propanol, 1,2-dimethoxyethane, diethyl ether, dichloroethane, tert-butylmethyl ether, 1-butanol, 2-butanol, t-butanol, 2-butanone, toluene, cyclohexane, heptane, hexane, $H_2O$, DMF, petroleum ethers and $CHCl_3$.

The most preferred solvent is acetonitrile.

Solvents may be used in combination with each other and preferred combinations include 10% HCl in $H_2O$, 10% $H_2O$ in DMF, 10% $H_2O$ in EtOH, 10% $H_2O$ in IPA, methanol and water in varying portions, methanol and water in varying portions with HCl, $CHCl_3$ and ethyl acetate in varying portions, EtOH and water in varying portions, IPA and water in varying portions, 1-propanol and water in varying portions and $CHCl_3$ and dioxane in varying portions.

More preferred solvent combinations include $CHCl_3$ and dioxane in varying portions, $CHCl_3$ and ethyl acetate in varying portions, methanol and water in varying portions with HCl and methanol and water in varying portions.

Commonly used chiral amine resolving agents include, without limitation, 1-phenylethyl amine, cinchonidine, cinchonine, quinidine, codeine, morphine, strychnine, brucine, quinine, ephedrine, amino butanol, dehydroabiethylamine, 2-phenyl glycinol, 1,2-cyclohexyldiamine, 1-naphthylethylamine, 2-amino-1-phenylpropane-1,3-diol, 4-chloro-1-phenylamine, N-(4-methoxybenzyl)-1-phenylethyl amine, fenchylamine, N-benzyl-1-phenylethyl amine, N-(4-dimethylaminobenzyl)-1-phenylethyl amine, 3-amino-2,2-dimethyl-3-phenylpropan-1-ol, sparteine, proline, serine, phenylalanine, lysine, threonine, valine, histidine, alanine, glutamic acid and glutamine, arginine, homo-arginine, N-$\alpha$-acetyl lysine, N-$\varepsilon$-acetyllysine and ornithine.

The most preferred chiral amine resolving agent is L-lysine.

Chiral amine resolving agents can be used in different stoichiometries to effect resolutions. The above resolving

agents can be used in a ratio of 1 part (I) to 10 parts of chiral amine (IV), more preferably in a ratio of 1 part (I) to 5 parts of chiral amine (IV), and most preferably in a ratio of 1 part (I) to 1 part of chiral amine (IV).

A variety of solvents may be useful to form diastereomeric salts (V) with chiral amines (IV). Preferred solvents will include ethyl acetate, dichloromethane, tetrahydrofuran, 1,4-dioxane, acetone, acetonitrile, MeOH, EtOH, IPA, 1-propanol, 1,2-dimethoxyethane, diethyl ether, dichloroethane, *tert*-butylmethyl ether, 1-butanol, 2-butanol, t-butanol, 2-butanone, toluene, cyclohexane, heptane, hexane, $H_2O$, DMF, petroleum ethers and $CHCl_3$.

References K. Akiba et. al. Bull. Chem. Soc. Jpn., 1974, 47(4), 935-937; R.A. Bartsch et. al., JACS, 2001, 123 (31), 7479 describe the decomposition of heterocyclic nitrosimines and may be applicable to the synthesis and resolution of glitazones. Therefore, an alternative method of synthesis/resolution, using pioglitazone as an example may be as shown in Scheme 3:

Scheme 3

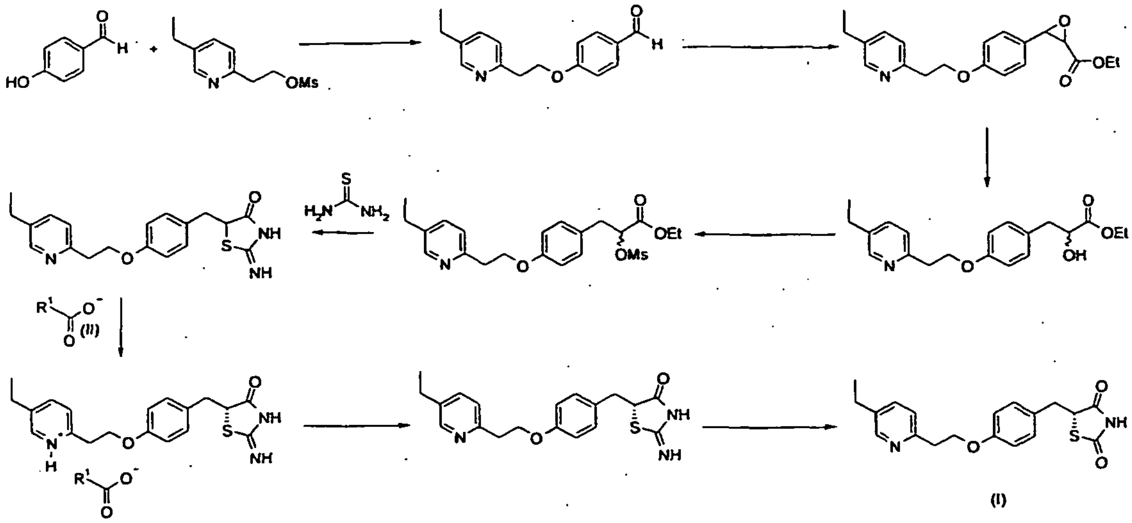

(II)

(I)

The final product may of course be recrystallised to increase enantiomeric purity.

Reference T. Sohda et al., Chem, Pharm. Bull., 1984, 32(11), 4460-5 describes the chiral synthesis of related compounds and may be applicable to the synthesis of other glitazones. Therefore, an alternative method of synthesis, using pioglitazone as an example may be as shown in Scheme 4:

Scheme 4

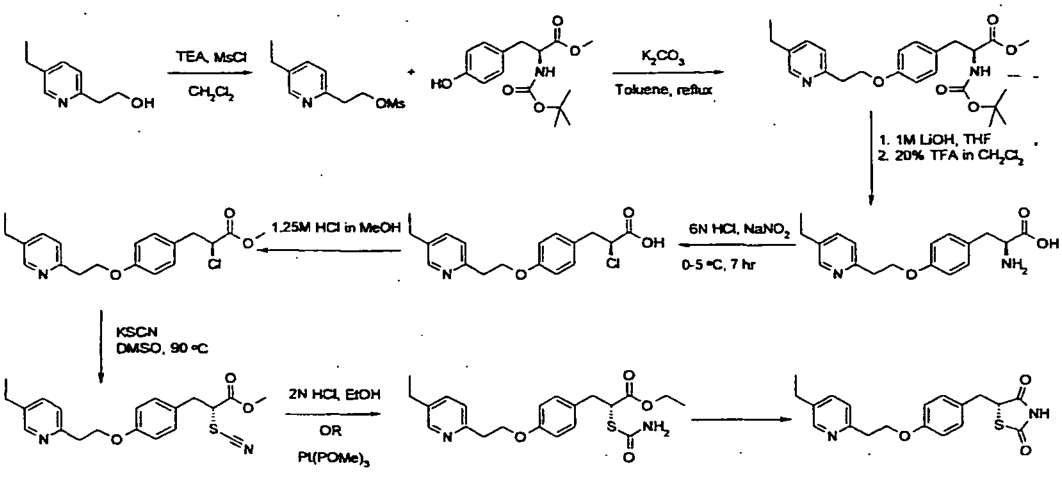


The final product may of course be recrystallised to increase enantiomeric purity.

Reference J.R. Falck et al., Bioorg.Med.Chem.Lett., 2008, 18, 1768-1771 describes the chiral synthesis of related compounds and may be applicable to the synthesis of glitazones. Therefore, an alternative method of synthesis, using pioglitazone as an example is as shown in scheme 5 (see also Example 13).

## Scheme 5

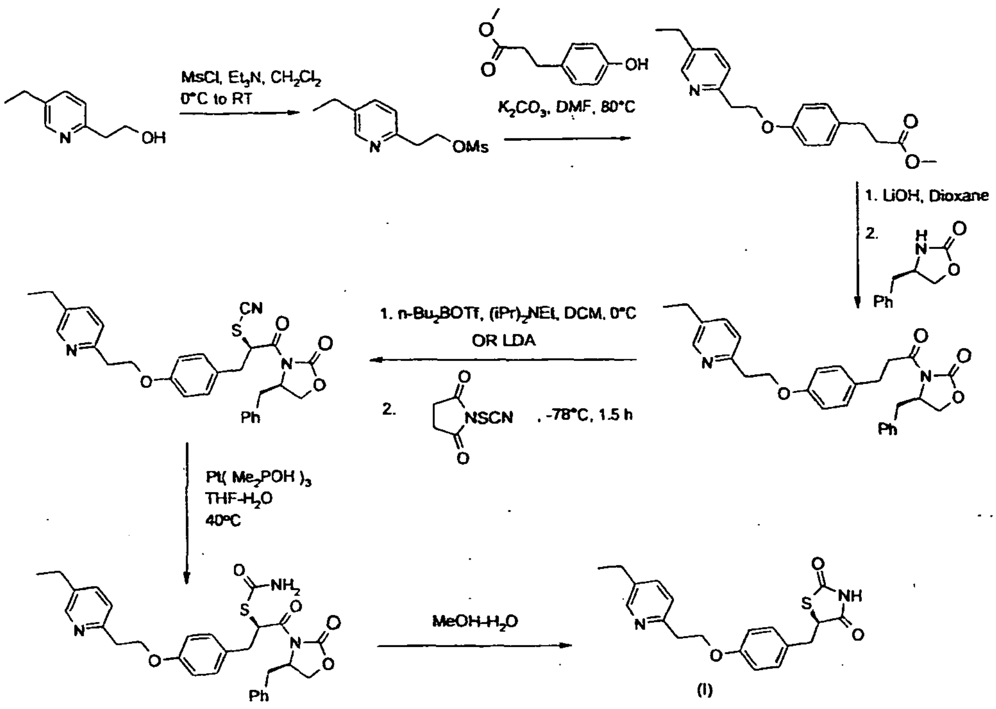


The final product may of course be recrystallised to increase enantiomeric purity.

In the case of rosiglitazone, reference J. Chem. Soc. Perkin Trans. I, 1994, 3319-3324 shows that 5-[1-{4-[3-(methyl-pyridin-2-yl-amino)-propoxy]-phenyl}-meth-(E)-ylidene]-thiazolidine-2,4-dione can be reduced using bio-catalysis. Yeast strains suggested to be useful for the purpose of biocatalysis are *Rhodotorula rubra* (or *mucilaginosa*) and *Rhodotorula glutinis.* Therefore an alternative preparation of glitazones may be, using pioglitazone as the example, as in Scheme 6:

## Scheme 6

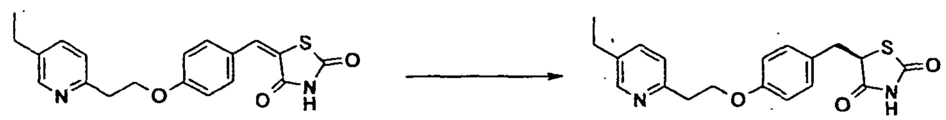

Alternatively, asymmetric hydrogenolysis of 5-[1-{4-[2-(5-ethyl-pyridin-2-yl)-e thoxy]-phenyl}-meth-(E)-ylidene]-thiazolidine-2,4-dione using Rhodium and Iridium catalysts in the presence of chiral ligands is a method known to those skilled in the art (See also Example 12 below).

The following Examples illustrate the preparation of pioglitazone and rosiglitazone enantiomers, and the biological results on which the present invention is based:

### CHEMICAL EXAMPLES

General Experimental Details

Abbreviations used in the experimental section:

c = concentration; h = hour, $H_2O$ = distilled water, HPLC = high performance liquid chromatography; LCMS = liquid chromatography mass spectrometry; MeOH = methanol; TFA = trifluoroacetic acid; DMSO = dimethyl sulphoxide; HCl = hydrogen chloride; EtOH = ethanol; IPA = isopropyl alcohol; EtOAc = ethyl acetate; THF = tetrahydrofuran; $NH_4Cl$ = ammonium chloride; LDA = lithium diisopropylamide; min = minutes; RT = room temperature; Rt = retention time; e.e. = enantiomeric excess; MP-Carbonate = macroporous triethylammonium methylpolystyrene carbonate (0.5% inorganic

antistatic agent). d.e. = diastereomeric excess; SL-W003-2 = (S)-1-[(S)-2-(2i-diphenylphosphinophenyl)ferrocenyl]ethyl-dicyclohexyl phosphine; $Rh(COD)_2BF_4$= bis(1,5-cyclooctadiene) Rhodium I tetrafluoroborate; pioglitazone = 5-{4-[2-(5-ethylpyridin-2-yl)ethoxy]benzyl}-1,3-thiazolidine-2,4-dione; L-DBTA = L-dibenzoyl tartaric acid

The nomenclature of structures was assigned using ACD Labs version 10.

NMR spectra were obtained on a Varian Unity Inova 400 spectrometer with a 5mm inverse detection triple resonance probe operating at 400MHz or on a Bruker Avance DRX 400 spectrometer with a 5mm inverse detection triple resonance TXI probe operating at 400MHz or on a Bruker Avance DPX 300 spectrometer with a standard 5mm dual frequency probe operating at 300MHz. Shifts are given in ppm relative to tetramethylsilane. Optical rotations were measured using an AA-10R automatic polarimeter with 5x25 mm jacketed sample cell. Asymmetric hydrogenolysis experiments were performed using Biotage Endeavor hydrogenation equipment.

All solvents and commercial reagents were used as received.

The Liquid Chromatography Mass Spectroscopy (LC/MS) and Liquid Chromatography systems used:

### Method 1

CHIRALPAK AD-H (250 x 30 mm, 5μm), elution with EtOH +0.05%TFA - flow rate 30 ml/min. Detection -In-line UV detection set at 250 nM wavelength

### Method 2

CHIRALPAK 1A (250 x 4.6 mm, 5μM), elution with EtOH +0.05%TFA - flow rate 0.7 ml/min. Detection - In-line DAD set at 280 nM wavelength

### Method 3

CHIRALCEL OD-RH (150 X 4.6 mm), elution with 90% MeOH + 10% $H_2O$ - flow rate 0.5 ml/min. Detection - In-line UV detection set at 254 nM wavelength

### Method 4

Waters Micromass ZQ2000 with a C18-reverse-phase column (100 × 3.0 mm Higgins Clipeus with 5 μm particle size), elution with A: water + 0.1 % formic acid; B: acetonitrile + 0.1 % formic acid. Gradient:

| Gradient - Time | flow mL/min | %A | %B |
|---|---|---|---|
| 0.00 | 1.0 | 95 | 5 |
| 1.00 | 1.0 | 95 | 5 |
| 15.00 | 1.0 | 5 | 95 |
| 20.00 | 1.0 | 5 | 95 |
| 22.00 | 1.0 | 95 | 5 |
| 25.00 | 1.0 | 95 | 5 |

Detection - MS, ELS, UV (100 μl split to MS with in-line UV detector). MS ionisation method - Electrospray (positive ion)

### Method 5

CHIRALPAK IA (250 x 21 mm, 5μm), elution with ethanol +0.2%TFA - flow rate 13 ml/min. Detection - In-line UV detection set at 220 nM wavelength

### Method 6

Chiral-AGP (150 x 4.0 mm, 5μM), elution with A: 86% 10 mM potassium dihydrogen phosphate buffer pH 7.0; B: 14% acetonitrile + 0.1 % formic acid - flow rate 0.8 ml/min. Detection - In-line DAD set at 254 nM wavelength

### Method 7

CHIRALPAK 1A (250 x 4.6 mm, 5μM), elution with A, 0.05% TFA in EtOH; B, heptane; D, IPA (A:B:D = 40:30:

30), - flow rate 0.7 ml/min. Detection - In-line DAD set at 225 nM wavelength Detection - MS, ELS, UV PDA. MS ionisation method - Electrospray (positive/negative ion)

**Method 8**

Waters Micromass ZQ2000 with a Acquity BEH or Acquity BEH Shield RP18 1.7uM 100 x 2.1 mm C18-reverse-phase column, elution with A: water + 0.1 % formic acid; B: acetonitrile + 0.1 % formic acid. Gradient:

| Gradient - Time | flow mL/min | %A | %B |
|---|---|---|---|
| 0.00 | 0.4 | 95 | 5 |
| 0.4 | 0.4 | 95 | 5 |
| 6.00 | 0.4 | 5 | 95 |
| 6.80 | 0.4 | 5 | 95 |
| 7.00 | 0.4 | 95 | 5 |
| 8.00 | 0.4 | 95 | 5 |

Detection - MS, ELS, UV PDA. MS ionisation method - Electrospray (positive/negative ion)

**Method 9**

Agilent 1100 series with a CHIRALPAK IA (150 x 4.6 mm, 5μm), elution with A: heptane, B: ethanol + 0.05%TFA - flow rate 0.5 ml/min. Detection - In-line polarimeter and UV detection set at 270 nM wavelength. Gradient:

| Gradient - Time | flow mL/min | %A | %B |
|---|---|---|---|
| 0.00 | 0.5 | 40 | 60 |
| 20.0 | 0.5 | 40 | 60 |
| 30.0 | 0.5 | 0 | 100 |
| 45.0 | 0.5 | 0 | 100 |

**Method 10**

Phenomenex Gemini C18-reverse-phase column (250 × 21.20 mm 5 μm particle size), elution with A: water + 0.1 % formic acid; B: methanol + 0.1 % formic acid. Gradient - 50% A/50% B to 5% A/95% B over 15 min - flow rate 18 mL/min. Detection - In-line UV detector set at 254 nM wavelength.

**Method 11**

Phenomenex Luna 3 micron C18(2) 30 x 4.6mm, elution with A: water + 0.1% formic acid; B: acetonitrile + 0.1 % formic acid. Gradient:

| Gradient - Time | flow mL/min | %A | %B |
|---|---|---|---|
| 0.00 | 2.0 | 95 | 5 |
| .50 | 2.0 | 95 | 5 |
| 4.50 | 2.0 | 5 | 95 |
| 5.50 | 2.0 | 5 | 95 |
| 6.00 | 2.0 | 95 | 5 |

Detection - MS, ELS, UV (200 μl/min split to MS with in-line HP1100 DAD detection). MS ionisation method - Electrospray (positive and negative ion)

All reactions were carried out under an atmosphere of nitrogen unless specified otherwise. Racemic rosiglitazone was used as a free base and racemic pioglitazone was used as a free base or HCl salt as indicated.

**Example 1**

**(5R)-5-{4-[2-(5-ethylpyridin-2-yl)ethoxy]benzyl}-1,3-thiazolidine-2,4-dione trifluoroacetate**

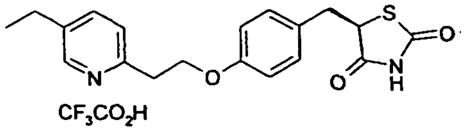

The *title compound* (480 mg) was isolated using method 1. LCMS (Method 4): Rt 6.00 min, m/z 357 [M-$CF_3CO_2H^+$] $[\alpha]_D^{25}$ +104° (c 1.0, MeOH). e.e. (Method 2) ≥ 98%, Rt 4.69 min. $^1$H NMR (400 MHz, DMSO-$d_6$): δ 12.02-11.88 (1H, bs), 8.68-8.60 (1 H, d, *J* 1.7), 8.32-8.23 (1 H, d, *J* 7.7), 7.90-7.82 (1 H, d, *J* 8.4), 7.14-7.06 (2 H, d, *J* 8.7), 6.85-6.78 (2 H, d, *J* 8.7), 4.85-4.78 (1 H, dd, *J* 4.4, 8.9), 4.35-4.27 (2 H, t, *J* 6.2), 3.40-3.34 (2 H, t, *J* 6.1), 3.28-3.21 (1 H, dd, *J* 4.3, 14.3), 3.05-2.97 (1 H, dd, *J* 9.0, 14.3), 2.77-2.67 (2 H, q, *J* 7.6), 1.22-1.14 (3 H, q, *J* 7.5).

**Example 2**

**(5S)-5-{4-[2-(5-ethylpyridin-2-yl)ethoxy]benzyl}-1,3-thiazolidine-2,4-dione trifluoroacetate**

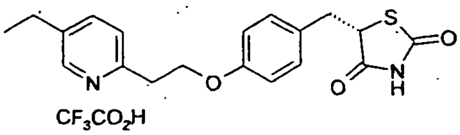

The *title compound* (674 mg) was isolated using method 1. LCMS (Method 4): Rt 6.01 min, m/z 357 [M-$CF_3CO_2H^+$]$[\alpha]_D^{25}$ -76° (c 1.0, MeOH). e.e. (Method 2) ≥ 98%, Rt 7.00 min. $^1$H NMR (400 MHz, DMSO-$d_6$): δ 12.02-11.88 (1H, bs), 8.68-8.60 (1 H, d, *J* 1.7), 8.32-8.23 (1 H, d, *J* 7.7), 7.90-7.82 (1 H, d, *J* 8.4), 7.14-7.06 (2 H, d, *J* 8.7), 6.85-6.78 (2 H, d, *J* 8.7), 4.85-4.78 (1 H, dd, *J* 4.4, 8.9), 4.35-4.27 (2 H, t, *J* 6.2), 3.40-3.34 (2 H, t, *J* 6.1), 3.28-3.21 (1 H, dd, *J* 4.3, 14.3), 3.05-2.97 (1 H, dd, *J* 9.0, 14.3), 2.77-2.67 (2 H, q, *J* 7.6), 1.22-1.14 (3 H, q, *J* 7.5).

**Example 3**

**(5R)-5-{4-[2-(5-ethylpyridin-2-yl)ethoxy]benzyl}-1,3-thiazolidine-2,4-dione**

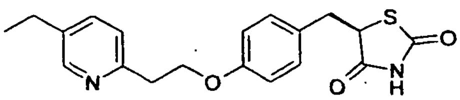

MP-Carbonate (389 mg, 1.06 mmol) was added to a solution of (5R)-5-{4-[2-(5-ethylpyridin-2-yl)ethoxy]benzyl}-1,3-thiazolidine-2,4-dione trifluoroacetate (100 mg, 0.21 mmol) in MeOH (100 mL) and stirred at RT for 2 h, filtered and the resin washed with MeOH (3 X 10 mL). The filtrate was concentrated in vacuo to afford the *title compound* (35mg, 47%). e.e. (Method 2) 92.90%, Rt 6.27 min. $^1$H NMR (400 MHz, DMSO-$d_6$): δ 12.44-11.11 (1 H, bs), 8.34-8.29 (1 H, d, *J* 1.9), 7.55-7.49 (1 H, dd, *J* 2.2, 7.9), 7.24-7.20 (1 H, d, *J* 7.8), 7.12-7.05 (2 H, d, *J* 8.6), 6.84-6.77 (2 H, d, *J* 8.6), 4.78-4.71 (1 H, dd, *J* 4.3, 9.1), 4.30-4.19 (1 H, d, *J* 4.3), 3.24-3.18 (2 H, d), 3.11-3.03 (2 H, t, *J* 6.6), 3.00-2.92 (1 H, dd, *J* 9.2, 14.2), 2.59-2.50 (2 H, q, *J* 7.6), 1.17-1.09 (3 H, t, *J* 7.7).

**Example 4**

**(5R)-5-{4-[2-(5-ethylpyridin-2-yl)ethoxy]benzyl}-1,3-thiazolidine-2,4-dione hydrochloride**

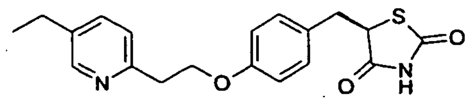

HCl

~1.25 M HCl in MeOH (0.33 mL, 0.33 mmol) was added to a suspension of (5R)-5-{4-[2-(5-ethylpyridin-2-yl) ethoxy]benzyl}-1,3-thiazolidine-2,4-dione (from Example 3) (30 mg, 0.084 mmol) in MeOH (5 mL) and stirred at RT for 1 h. The solvent was removed in vacuo to afford the *title compound* (32.4 mg, 100%). LCMS (Method 4): Rt 5.95 min, m/z 357 [M-HCl$^+$]. e.e (Method 3) 93.2%, Rt 12.10 min. Stereochemisty at C-5 was assigned (R) configuration by single crystal X-ray diffraction analysis. $[\alpha]_D^{24}$ +108° (c 1.0, MeOH). $^1$H NMR (400 MHz, DMSO-d$_6$): δ 12.03-11.88 (1 H, bs), 8.68-8.62 (1 H, d, *J* 1.7), 8.34-8.25 (1 H, d, *J* 7.9), 7.91-7.83 (1 H, d, *J* 8.3), 7.14-7.05 (2 H, d, *J* 8.7), 6.86-6.77 (2 H, d, *J* 8.7), 4.85-4.77 (1 H, dd, *J* 4.3, 8.9), 4.38-4.28 (2 H, t, *J* 6.0), 3.42-3.36 (2 H, t, *J* 6.2), 3.28-3.20 (1 H, dd, *J* 9.0, 14.2), 3.06-2.96 (1 H, dd, *J* 9.0, 14.2), 2.77-2.67 (2 H, q, *J* 7.7), 1.23-1.15 (3 H, t. *J* 7.7). Subsequent recrystallisations using MeOH-EtOAc or MeOH-$Et_2O$ gave the *title compound* with an e.e. >97%.

**Example 5**

**(5S)-5-{4-[2-(5-ethylpyridin-2-yl)ethoxy]benzyl}-1,3-thiazolidine-2,4-dione**

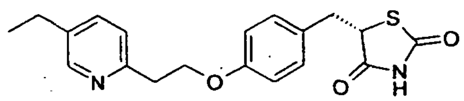

The title compound (28 mg, 37%) was prepared using a method analogous to that outlined in Example 3 starting from (5S)-5-{4-[2-(5-ethylpyridin-2-yl)ethoxy]benzyl}-1,3-thiazolidine-2,4-dione trifluoroacetate. e.e. (Method 2) 90.9%, Rt 9.21 min $^1$H NMR (400 MHz, DMSO-*d*$_6$): δ 12.44-11.11 (1 H, bs), 8.34-8.29 (1 H, d, *J* 1.9), 7.55-7.49 (1 H, dd, *J* 2.2, 7.9), 7.24-7.20 (1 H, d, *J* 7.8), 7.12-7.05 (2 H, d, *J* 8.6), 6.84-6.77 (2 H, d, *J* 8.6), 4.78-4.71 (1 H, dd, *J* 4.3, 9.1), 4.30-4.19 (1 H, d, *J* 4.3), 3.24-3.18 (2 H, d), 3.11-3.03 (2 H, t, *J* 6.6), 3.00-2.92 (1 H, dd, *J* 9.2, 14.2), 2.59-2.50 (2H, q, *J* 7.6), 1.17-1.09 (3 H, t, *J* 7.7).

**Example 6**

**(5S)-5-{4-[2-(5-ethylpyridin-2-yl)ethoxy]benzyl}-1,3-thiazolidine-2,4-dione hydrochloride**

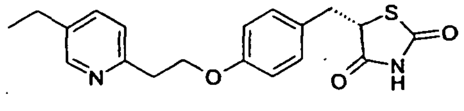

HCl

The *title compound* (25.7 mg, 100%) was prepared using a method analogous to that outlined in Example 4 starting from (5S)-5-{4-[2-(5-ethylpyridin-2-yl)ethoxy]benzyl}-1,3-thiazolidine-2,4-dione (from Example 2). LCMS (Method 4): Rt 5.94 min, m/z 357 [M-HCl$^+$]. e.e. (Method 3) 92.7%, Rt 13.25 min. Stereochemisty at C-5 was assigned (S) configuration by single crystal X-ray diffraction analysis. $[\alpha]_D^{23}$-104° (*c* 1.0, MeOH $^1$H NMR (400 MHz, DMSO-*d*$_6$): δ 12.03-11.88 (1 H, bs), 8.68-8.62 (1 H, d, *J* 1.7), 8.34-8.25 (1 H, d, *J* 7.9), 7.91-7.83 (1 H, d, *J* 8.3), 7.14-7.05 (2 H, *d*, *J* 8.7), 6.86-6.77 (2 H, *d*, *J* 8.7), 4.85-4.77 (1 H, *dd*, *J* 4.3, 8.9), 4.38-4.28 (2 H, t, *J* 6.0), 3.42-3.36 (2 H, t, *J* 6.2), 3.28-3.20

(1 H, dd, *J* 9.0, 14.2), 3.06-2.96 (1 H, dd, *J* 9.0, 14.2), 2.77-2.67 (2 H, q, *J* 7.7), 1.23-1.15 (3 H, t, *J* 7.7). Subsequent recrystallisations using MeOH-EtOAc or MeOH-$Et_2O$ gave the *title compound* with an e.e. >97%.

**Example 7**

**(5R)-5-(4-{2-[methyl(pyridin-2-yl)amino]ethoxy}benzyl}-1,3-thiazolidine-2,4-dione trifluoroacetate**

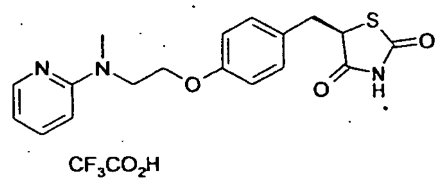

The *title compound* (149 mg) was isolated using method 5. Rt 7.14 min. $^1$H NMR (400 MHz, DMSO-$d_6$): δ 12.04-11.86 (1 H, s), 7.99-7.94 (1 H, dd, *J* 1.1, 6.2), 7.92-7.83 (1 H, t, *J* 6.6), 7.27-7.15 (1 H, d, *J* 8.7), 7.13-7.05 (2 H, d, *J* 8.6), 6.88-6.82 (1 H, *t*, *J* 6.6), 6.81-6.76 (2 H, d, *J* 8.7), 4.83-4.78 (1 H, dd, *J* 4.4, 8.8), 4.18-4.12 (2 H, t, *J* 5.3), 3.99-3.94 (2 H, t, *J* 5.3), 3.27-3.20 (1 H, dd, *J* 4.2, 14.4), 3.18 (3 H, s), 3.05-2.97 (1 H, dd, *J* 8.9, 14.6).

**Example 8**

**(5R)-5-(4-{2-[methyl(pyridin-2-yl)amino]ethoxy}benzyl)-1,3-thiazolidine-2,4-dione hydrochloride**

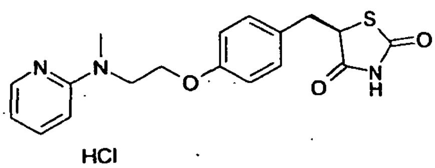

The *title compound* (38 mg, 64%) was prepared using method analogous to that outlined in Example 3 and 4 starting from (5R)-5-{4-[2-methyl-2-pyridylamino)ethoxy]benzyl}-1,3-thiazolidine-2,4-dione trifluoroacetate. $[\alpha]_D^{26}$ +100° (c 1.0, MeOH). LCMS (Method 4): Rt 5.41 min, m/z 358 [M-HCl$^+$]. e.e. (Method 6) 85.7%, Rt 8.03 min. $^1$H NMR (400 MHz, DMSO-$d_6$): δ 12.09-11.81 (1 H, s), 7.98-7.94 (1 H, dd, *J* 1.1, 6.3), 7.93-7.82 (1 H, m), 7.31-7.14 (1 H, bs), 7.13-7.05 (2 H, d, *J* 8.5), 6.90-6.82 (1 H, *t*, *J* 6.7), 6.80-6.76 (2 H, d, *J* 8.5), 4.84-4.78 (1 H, dd, *J* 4.4, 8.9), 4.19-4.12 (2 H, t, *J* 5.2), 4.02-3.94 (2 H, t, *J* 5.2), 3.27-3.21 (1 H, dd, *J* 4.4, 10.1), 3.20 (3 H, s), 3.05-2.97 (1 H, dd, *J* 9.0, 14.2).

R-enantiomer with greater than 90% e.e. can be obtained using literature procedures, *J. Chem. Soc. Perkin Trans.* 1. **1994**, 3319-3324.

**Example 9**

**(5S)-5-(4-{2-[methyl(pyridin-2-yl)amino]ethoxy}benzyl)-1,3-thiazolidine-2,4-dione hydrochloride monohydrate**

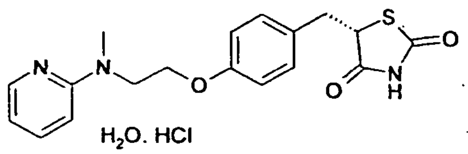

The *title compound* (123 mg) was prepared using literature procedures, *J. Chem. Soc. Perkin Trans. 1.* **1994,** 3319-3324. $[\alpha]_D^{23}$-100° (*c* 1.0, MeOH). LCMS (Method 4): Rt 5.44 min, m/z 358 [M-HCl$^+$]. e.e. (Method 6) 92.7%, Rt 8.99 min. Stereochemisty at C-5 was assigned (S) configuration by single crystal X-ray diffraction analysis. $^1$H NMR (400 MHz, DMSO-$d_6$): δ 12.01-11.88 (1 H, s), 7.98-7.94 (1 H, dd, *J* 1.4, 6.1), 7.93-7.86 (1 H, t, *J* 7.7), 7.31-7.18 (1 H, m), 7.12-7.05 (2 H, d, *J* 8.7), 6.90-6.83 (1 H, *t*, *J* 6.3), 6.81-6.75 (2 H, d, *J* 8.7), 4.83-4.78 (1 H, dd, *J* 4.5, 8.8), 4.19-4.13

(2 H, t, *J* 5.1), 4.02-3.96 (2 H, t, *J* 5.1), 3.26-3.22 (1 H, m), 3.21 (3 H, s), 3.05-2.97 (1 H, dd, *J* 8.8, 14.0).

**Example 10**

**(5R)-5-{4-[2-(5-ethylpyridin-2-yl)ethoxy]benzyl}-1,3-thiazolidine-2,4-dione(-)-O,O'-dibenzoyl-L-tartrate**

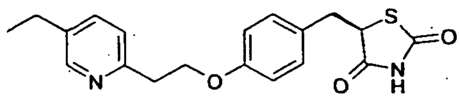

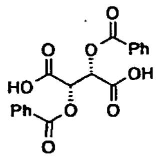

**10a.** To a mixture of (5R)-5-{4-[2-(5-ethylpyridin-2-yl)ethoxy]benzyl}-1,3-thiazolidine-2,4-dione hydrochloride (50mg) (example 4) and (-)-dibenzoyl-L-tartaric acid (50mg) was added MeOH (1.5 mL). The clear solution was rapidly stirred whilst adding $H_2O$ dropwise until a cloudiness persisted. The reaction was allowed to stand at ambient temperature over 48h and the solid collected by fitration to give the *title compound* (43mg). (Method 7) 99.01% Rt 10.83 min, 0.98% Rt 15.83 min; d.e. 98.03%

10b. A slurry of (-)-dibenzoyl-L-tartaric acid (1.0g, 2.79 mmol) in $H_2O$ (20mL) was stirred at ambient temperature and a solution of 5-{4-[2-(5-ethylpyridin-2-yl)ethoxy]benzyl}-1,3-thiazolidine-2,4-dione hydrochloride (1.01g, 2.57mmol) in MeOH (20mL) was added over 5 min. When addition was complete, the product from Example 10a (5mg) was added and the reaction allowed to stir for 93h. The reaction was filtered and the solid dried to give the *title compound* (0.863g). (Method 7) 79.72%, Rt 10.82 min.; 20.27%, Rt 15.14 min.; d.e. 59.45%.

10c. The product from Example 10b (0.863g) was dissolved in MeOH (8.5 mL) containing 1M HCl (1.21 mL) and $H_2O$ (5 mL) added dropwise. The product from Example 10a (1 mg) was added followed by dropwise addition of $H_2O$ (2.3 mL). The reaction was allowed to stir for 22h, filtered, the solid washed with $H_2O$-MeO (2:1, 3 mL) and dried at 40°C under high vacuum to give the *title compound* (0.582g). (Method 7) 93.2%, Rt 10.82 min.; 6.8%, Rt 15.14 min.; d.e. 86.4%.

**10d**. The product from Example 10c (0.582g) was dissolved in MeOH (5.5 mL) containing 1M HCl (0.795 mL) and $H_2O$ (2 mL) added dropwise. The product from Example 10a (1 mg) was added followed by dropwise addition of $H_2O$ (3.5 mL). The reaction was allowed to stir for 22h, filtered, the solid washed with $H_2O$-MeOH (2:1, 3 mL) and dried at 40°C under high vacuum to give the *title compound* (0.453g). (Method 7) 97.3%, Rt 10.65 min.; 2.7%, Rt 14.83 min.; d.e. 94.6%. $^1$H NMR (400 MHz, DMSO-$d_6$): 14.25-13.60 (bs, 1H, $D_2O$ exchangeable), 12.05-12.00 (bs, 1H, $D_2O$ exchangeable), 8.37 (d, *J* = 2.0 Hz, 1H), 8.02 (d, *J* = 7.6 Hz, 4H), 7.73 (t, *J* = 7.6 Hz, 2H), 7.60 (t, *J* = 7.6 Hz, 5H), 7.29 (d, *J* = 8.0 Hz, 1H), 7.13 (d, *J* = 8.8 Hz, 2H), 6.86 (d, *J* = 8.8 Hz, 2H), 5.88 (s, 2H), 4.86 (q, *J* = 4.4 Hz, 1H), 4.30 (t, *J* = 6.8 Hz, 2H), 3.30 (dd, *J* = 4.0 & 10.0 Hz, 1H), 3.13 (t, *J* = 6.8 Hz, 2H), 3.04 (dd, *J* = 5.2 & 9.2 Hz, 1H), 2.60 (q, *J* = 7.6 Hz, 2H), 1.17 (t, *J* = 7.6 Hz, 3H).

**Example 11 1**

**(5R)-5-{4-[2-(5-ethylpyridin-2-yl)ethoxy]benzyl}-1,3-thiazolidine-2,4-dione hydrochloride**

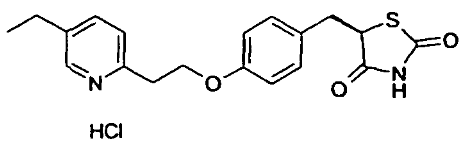

**11a.** The product from Example 10d was dissolved in MeOH (2.25 mL) containing 37% HCl (0.134 mL) at 35

°C. The solution was filtered, EtOAc (9 mL) was poured into the stirred solution and the mixture stirred for 20 min. The white solid was collected by filtration, washed with EtOAc and dried at 30 °C under high vacuum to give the *title compound* (0.181 g). (Method 7) 98.3%, Rt 10.65 min.; 1.7%, Rt 14.83 min e.e. 96.6%. LCMS (Method 8): Rt 2.90 min 99.39%, m/z 357 [$MH^+$-HCl]. LCMS (Method 11) Rt 2.91 min, m/z 357 [$MH^+$-HCl]. $^1$H NMR (400 MHz, DMSO-$d_6$): δ 12.0 (1 H, s), 8.70 (1 H, d, *J* 1.7 Hz), 8.36 (1 H, bd, *J* 8.3 Hz), 7.93 (1 H, d, *J* 8.2 Hz), 7.15, 6.87 (4 H, A2B2q, *J* 8.7 Hz), 4.86 (1 H, dd, *J* 4.4, 8.9 Hz), 4.38 (2 H, t, *J* 6.3 Hz), 3.44 (2 H, t, *J* 6.2 Hz), 3.29 (1 H, dd, *J* 4.3, 14.2 Hz), 3.06 (1 H, dd, *J* 9.0, 14.3 Hz), 2.78 (2 H, q, *J* 7.6 Hz), 1.23 (3 H, t, *J* 7.6 Hz).

11b. The product from Example 10c (1g) could also be reacted as described in Example 11a to give the *title compound* (473mg). (Method 7) 95.6%, Rt 10.65 min.; 4.3%, Rt 14.83 min e.e. 91.3%. All other characterisation data was the same as Example 11a.

**Example 12**

**(5R)-5-{4-[2-(5-ethylpyridin-2-yl)ethoxy]benzyl}-1,3-thiazolidine-2,4-dione**

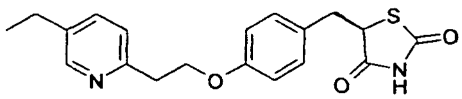

A stock solution of the $Rh(COD)_2BF_4$ (10.6 mg, 26.1 μmol) in DCM (9.5 mL) was made in a glove box. Ligand SL-W003-2 (8.4 mg, 12.5 μmol), was weighted into a vial and 500 μL of the $Rh(COD)_2BF_4$ stock solution was added. A solution of 5-[1-{4-[2-(5-ethyl-pyridin-2-yl)-ethoxy]-phenyl}-meth-(E)-ylidene]-thiazolidine-2,4-dione (4.5 mL of a stock solution containing 78.2 mg in 35 mL MeOH) was added to the reaction vial containing the catalyst solution. The vial was purged with $N_2$ (5 x) and $H_2$ (5 x) and finally charged with 25 bar of $H_2$. After 18h the $H_2$ was released and the reaction vial was purged with $N_2$ (3 x). A sample of the reaction vial was taken and diluted with an equal volume of EtOH containing 1% formic acid. e.e. (Method 9) 75%, Rt 36.11 min.

**Example 13**

**(5R)-5-{4-[2-(5-ethylpyridin-2-yl)ethoxy]benzyl}-1,3-thiazolidine-2,4-dione Hydrochloride**

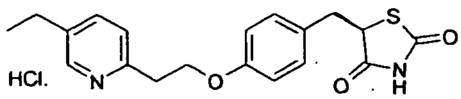

13a. Methanesulfonic acid 2-(5-ethyl-pyridin-2-yl)-ethyl ester

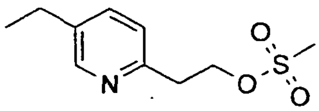

To a solution of 2-(5-ethyl-pyridine-2-yl)-ethanol (25.34 g) and triethylamine (46.7 mL) in DCM (130 mL) at 0 °C under nitrogen was added methane sulfonyl chloride (15.56 mL), then the reaction mixture was allowed to warm to room temperature and stirred overnight. A further portion of methane sulfonyl chloride (3.88 mL) was added and the reaction stirred for 30 min. The reaction was diluted with DCM and washed with $H_2O$ and brine. The organic layer was dried ($MgSO_4$) and the solution concentrated to give the title compound as a red oil (38.24 g). $^1$H NMR (400 MHz, $CDCl_3$ ): δ 8.39 (d, *J* = 2.2 Hz, 1H), 7.47 (dd, *J* = 7.7, 2.3Hz, 1H), 7.14 (d, *J* = 7.7 Hz, 1H), 4.64 (t, *J* = 6.5 Hz, 2H), 3.19 (t, *J* = 6.5 Hz, 2H), 2.90 (s, 3H), 2.64 (q, *J* = 7.6 Hz, 2 H), 1.25 (t, *J* = 7.6 Hz, 3H).

13b. 3-{4-[2-(5-Ethyl-pyridin-2-yl)-ethoxy]-phenyl}-propionic acid methyl ester

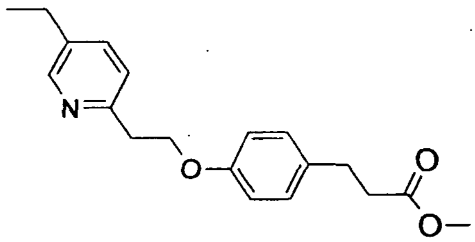

The product from Example 13a (12.72 g) in toluene (50 mL) was added dropwise to a stirred mixture of methyl-3-(4-hydroxyphenyl)propionate (10 g) and potassium carbonate (23.01 g) in toluene (180 mL). The reaction was heated to reflux for 23 h, then left at room temperature for 90 h. The reaction mixture was treated with $H_2O$ and extracted with three portions of diethyl ether. The combined extracts were washed twice with $H_2O$, once with brine, dried and evaporated. The residue was purified by chromatography eluting with 20-40% EtOAc in petroleum ether (bp = 40-60 °C) to give the title product as a yellow solid (10.73 g). $^1$H NMR (400 MHz, $CDCl_3$ ): δ 8.39 (d, *J* = 2.1 Hz, 1H), 7.44 (dd, *J* = 7.8, 2.4Hz, 1H), 7.18 (d, J=7.8 Hz, 1H), 7.08, 6.83 ($A_2B_2$q, J = 8.6 Hz, 4H), 4.31 (t, J = 6.7 Hz, 2H), 3.66 (s, 3H), 3.22 (t, *J* = 6.7 Hz, 2H), 2.87 (t, *J* = 7.6 Hz, 2H), 2.66-2.55 (m, 4H), 1.24 (t, *J* = 7.6 Hz, 3H).

13c. 3-{4-[2-(5-Ethyl-pyridin-2-yl)-ethoxy]-phenyl}-propionic acid

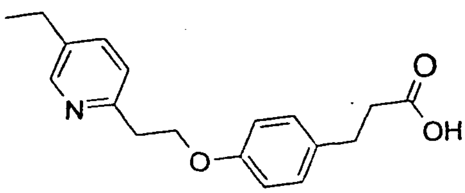

The product from Example 13b (10.73 g) was dissolved in 1,4-dioxan/ water (350/100 mL), lithium hydroxide monohydrate (4.3 g) added, and the reaction stirred overnight. The dioxan was removed by evaporation under reduced pressure, the suspension was diluted with $H_2O$ and treated with 2.0 M HCl until the pH was 6-7. The solid was filtered and dried to give the title compound (9.57 g).$^1$H NMR (400 MHz, $CDCl_3$ ): δ 8.41 (d, *J* = 2.3 Hz, 1H), 7.52 (dd, *J* = 7.9, 2.3Hz, 1H), 7.25 (d, *J* = 7.9 Hz, 1H), 7.11, 6.81 ($A_2B_2$q, *J* = 8.6 Hz, 4H), 4.27 (t, *J* = 6.6 Hz, 2H), 4.0 (bs, 2H, COOH+water) 3.24 (t, *J* = 6.6 Hz, 2H), 2.90 (t, *J* = 7.6 Hz, 2H), 2.68-2.59 (m, 4H), 1.24 (t, *J* = 7.6 Hz, 3H).

13d. (R)-4-Benzyl-3-(3-{4-[2-(5-ethyl-pyridin-2-yl)-ethoxy]-phenyl}-propionyl)-oxazolidin-2-one

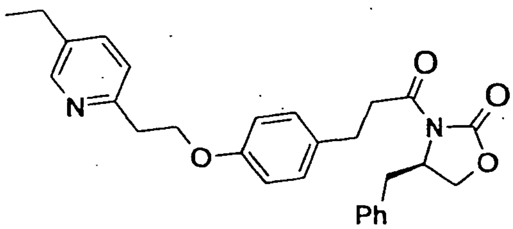

A suspension of the product from Example 13c (9.0 g) and (R)-(+)-4-benzyloxazolidinone (2.67 g) in triethylamine (8.38 mL) and toluene (90 mL) was heated to 80 °C. Pivaloyl chloride (3.71 mL) was added dropwise maintaining the temperature between 80 and 85 °C. The reaction was then heated to reflux for 22.5 h. The reaction was allowed to cool to room temperature, the reaction partitioned between $H_2O$-EtOAc and the aqueous layer was extracted twice with EtOAc and the combined organics dried ($MgSO_4$) and concentrated. The crude product was purified by chromatography on silica gel eluting with 0-40% EtOAccyclohexane to give the title product as a white solid (2.06 g). $^1$H NMR (300 MHz, $CDCl_3$): δ 8.39 (d, *J* = 2.2 Hz, 1H), 7.45 (dd, *J* = 7.9, 2.2Hz, 1H), 7.36-7.24 (m, 3H), 7.21-7.12 (m, 5H), 6.84 (d, *J* = 8.6 Hz, 2H), 4.65 (m, 1H), 4.32 (t, *J* = 6.6 Hz, 2H), 4.16 (m, 2H), 3.33-3.12 (m, 5H), 3.04-2.87 (m, 2H), 2.74 (dd, *J* = 9.5, 13.4 Hz, 1H), 2.63 (q, *J* = 7.6 Hz, 2H), 1:24 (t, *J* = 7.6 Hz, 3H).

13e. (R)-4-Benzyl-3-((R)-3-{4-[2-(5-ethyl-pyridin-2-yl)-ethoxy]-phenyl}-2-thiocyanato-propionyl)-oxazolidin-2-one

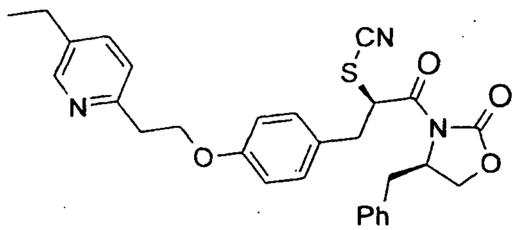

To LDA (0.528 mmol) in THF/hexanes (3/0.3 mL) at -78 °C under argon was added dropwise the product from Example 13d (0.20 g) in THF (4 mL). After 30 min N-thiocyanatosuccinimide (JACS, 2004, 126, 10216-7) (0.137 g) in THF (2 mL) was added dropwise. After a further 110 min at -78 °C the reaction was quenched with sat aqueous $NH_4Cl$ (5 mL) and allowed to warm to room temperature. The mixture was extracted with three 15 mL portions of EtOAc, the combined organic layers were dried ($Na_2SO_4$) and evaporated under reduced pressure to low volume. Toluene (20 mL) was added and evaporated under reduced pressure. The residue was dissolved in DCM (3 mL) and stored at -20 °C for 16 h. The soluble material was purified by chromatography on silica gel eluting with 20-50% EtOAc in cyclohexane, repurifying impure fractions by the same method, to give the title product and succinimide in 1:2.4 molar ratio as a colourless semi solid (0.121 g). $^1H$ NMR (300 MHz, $CDCl_3$ ): δ 8.60 (bs, 1H succinimide) 8.40 (d, *J* = 2.3 Hz, 1H), 7.47 (dd, *J* = 7.8, 2.3Hz, 1H), 7.39-7.12 (m, 8H), 6.85 (d, *J* = 8.7 Hz, 2H), 5.07 (dd, *J* = 8.1; 7.2 Hz, 1H), 4.63 (m, 1H), 4.32 (t, *J* = 6.7 Hz, 2H), 4.24-4.11 (m, 2H), 3.47 (dd, *J* = 14.0, 8.0 Hz, 1H), 3.33 (dd, *J* = 13.4, 3.2 Hz, 1H), 3.28-3.16 (m, 3H), 2.84 (dd, *J* = 9.3, 13.4 Hz, 1H), 2.76 (s, 4H succinimide) 2.63 (q, *J* = 7.6 Hz, 2H), 1.24 (t, *J* = 7.6 Hz, 3H).

13f. Thiocarbamic acid S-((R)-2-((R)-4-benzyl-2-oxo-oxazolidin-3-yl)-1-{4-[2-(5-ethyl-pyridin-2-yl)-ethoxy]-benzyl}-2-oxo-ethyl) ester

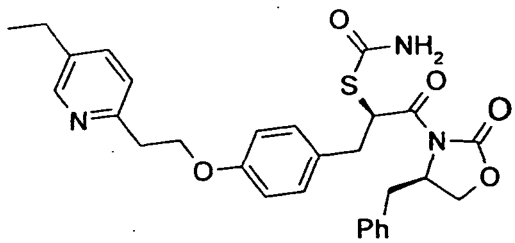

The product from Example 13e (51 mg) was dissolved in THF/water (2/1 ml) under argon and treated with platinum tris(dimethylphosphine oxide) (*Tet. Lett.,* 2002, 43, 8121) (4 mg). The reaction was warmed to 40 °C for 2 h then allowed to cool to room temperature. EtOAc (10 mL) was added, the organic phase dried ($Na_2SO_4$), concentrated and purified by chromatography on silica gel (2 g) eluting with 40-70% EtOAc in cyclohexane to give the title product and succinimide in 1:3.3 molar ratio as a colourless semi solid (0.029 g). $^1H$ NMR

(400 MHz, $CDCl_3$): δ 8.66 (bs, 1H succinimide) 8.39 (d, *J* = 2.0 Hz, 1H), 7.47 (dd, *J* = 7.9, 2.2 Hz, 1H), 7.37-7.17 (m, 6H), 7.17, 6.81 ($A_2B_2q$ *J* = 8.5 Hz, 4H), 5.74 (t, *J* = 7.9 Hz, 1H), 5.63 (bs, 2H), 4.53 (m, 1H), 4.30 (t, *J* = 6.7 Hz, 2H), 4.08 (dd, *J* = 8.9, 2.5 Hz, 1H), 3.95 (dd, *J* = 8.9, 7.8 Hz, 1H), 3.31 (dd, *J* = 13.5, 3.1 Hz, 1H), 3.26-3.18 (m, 3H), 2.96 (dd, *J* = 13.5, 8.1 Hz, 1H), 2.74 (s, 4H succinimide), 2.72 (dd, *J* = 13.5, 9.8 Hz, 1H), 2.63 (q, *J* = 7.6 Hz, 2H), 1.24 (t, *J* = 7.6 Hz, 3H).

**13g. (5R)-5-{4-[2-(5-ethylpyridin-2-yl)ethoxy]benzyl}-1,3-thiazolidine-2,4-dione hydrochloride**

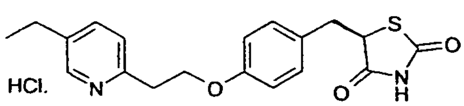

The product from Example 13f was dissolved in MeOH (10 mL) and $H_2O$ (10 ml) added to give a cloudy solution. This was left at room temperature for 2.5h then 1 M HCl (0.15 mL) added. The solution was evaporated to dryness and the residue taken up in MeOH (0.1 mL) containing concentrated HCl (0.0015 mL). EtOAc (5 mL) was added and the solution concentrated slightly under reduced pressure, causing a white solid to precipitate. The solution was removed and the solid (7 mg) washed with EtOAc. This material was purified by preparative HPLC (Method 10), and the fractions

containing the first eluting component combined and treated with 1M HCl (1 mL) and evaporated to dryness to give the *title product* and succinimide in a 1:0:0.19 molar ratio as a colourless gum (0.002 g). (Method 7) 99.175%, Rt 10.65 min.; 0.825%, Rt 14.83 min.; e.e. 98.35%. LCMS (Method 11) Rt 2.9 min, m/z 357 [$MH^+$]. $^1H$ NMR (300 MHz, $d_4$-MeOH): 8.62 (bd, 1H), 8.42 (dd, *J* = 8.2, 2.0 Hz, 1H), 7.98 (d, *J* = 8.2 Hz, 1H), 7.16, 6.85 ($A_2B_{2q}$, *J* = 8.7 Hz, 4H), 4.68 dd, *J* = 8.7, 4.2 Hz, 1H), 4.38 (t, *J* = 5.8 Hz, 2H), 3.34 (m obscured, 1H), 3.11 (dd, *J* = 14.3, 8.8 Hz, 1H), 2.87 (q, *J* = 7.6 Hz, 2H), 2.68 (s, 4H succinimide),1.33 (t, *J* = 7.6 Hz, 3H).

Biological Results:

**Example 14**

*Pre-clinical mouse model of COPD inflammation - Tobacco smoke induced pulmonary inflammation.*

Previous studies have established that the number of inflammatory cells recovered in the bronchoalveolar lavage (BAL) is significantly elevated 24 h following the final Tobacco Smoke (TS) exposure of 4 or 11 consecutive daily TS exposures, this time point was used in the studies reported here.

Protocols for the exposure of mice to TS, obtaining bronchoalveolar lavage (BAL), preparation of cytospin slides for differential cell counts are as outlined below.

*Exposure of mice to TS daily for 4 or 11 consecutive days*

In this exposure protocol, mice were exposed in groups of 5 in individual clear polycarbonate chambers (27 cm x 16 cm x 12 cm). The TS from the cigarettes was allowed to enter the exposure chambers at a flow rate of 100 ml/min. In order to minimise any potential problems caused by repeated exposure to a high level of TS (6 cigarettes), the exposure of the mice to TS was increased gradually over the exposure period to a maximum of 6 cigarettes. The exposure schedule used for 4 days was as follows:

| | |
|---|---|
| Day 1: 4 cigarettes | (approximately 32 min exposure) |
| Day 2: 4 cigarettes | (approximately 32 min exposure) |
| Day 3: 6 cigarettes | (approximately 48 min exposure) |
| Day 4: 6 cigarettes | (approximately 48 min exposure) |

The exposure schedule used for 11 days exposure was as follows:

| | |
|---|---|
| Day 1: 2 cigarettes | (approximately 16 min exposure) |
| Day 2: 3 cigarettes | (approximately 24 min exposure) |
| Day 3: 4 cigarettes | (approximately 32 min exposure) |
| Day 4: 5 cigarettes | (approximately 40 min exposure) |
| Day 5 to 11: 6 cigarettes | (approximately 48 min exposure) |

A further group of mice were exposed to air on a daily basis for equivalent lengths of time as controls (no TS exposure).

*Bronchoalveolar lavage (BAL) analysis*

Bronchoalveolar lavage was performed as follows: the trachea was cannulated using a Portex nylon intravenous cannula (pink luer fitting) shortened to approximately 8 mm. Phosphate buffered saline (PBS) was used as the lavage fluid. A volume of 0.4 ml was gently instilled and withdrawn 3 times using a 1ml syringe and then placed in an Eppendorf tube and kept on ice prior to subsequent determinations.

Cell counts:

Lavage fluid was separated from cells by centrifugation and the supernatant decanted and frozen for subsequent analysis. The cell pellet was re-suspended in a known volume of PBS and total cell numbers calculated by counting a stained (Turks stain) aliquot under a microscope using a haemocytometer.

Differential cell counts were performed as follows:

The residual cell pellet was diluted to approximately $10^5$ cells per ml. A volume of 500 μl was placed in the funnel of a cytospin slide and centrifuged for 8 min at 800 rpm. The slide was air dried and stained using 'Kwik-Diff' solutions (Shandon) as per the proprietary instructions.
When dried and cover-slipped, differential cells were counted using light microscopy. Up to 400 cells were counted by unbiased operator using light microscopy. Cells were differentiated using standard morphometric techniques.

Drug Treatment

Rodents such as mice and rats are obligate nasal breathers thus oral delivery of test materials (such as therapeutic agents) for inhalation will not produce good lung exposure. As a consequence, delivery of therapeutic agents to the lungs in rodents is generally achieved by intra-nasal, intra-tracheal or inhalation by whole body aerosol exposure in a chamber.
The chamber method utilises large amounts of test material and is generally reserved for inhalation toxicology studies rather than pharmacological efficacy studies. Intra-tracheal administration is a very efficient delivery method as almost all of the test material is delivered to the lungs, but this is quite an invasive technique. For studies in the mouse particularly, it is also quite technically demanding as the diameter of the trachea is quite small. The intranasal route is less invasive than the intra-tracheal route and so is particularly suitable for repeat dosing studies such as the 4-11 day mouse model described below. Following intranasal administration ~50% of the dose administered is delivered to the lungs (Eyles JE, Williamson ED and Alpar HO. 1999, Int J Pharm, 189(1):75-9).
As a surrogate route for oral inhalation, mice were dosed intra-nasally with vehicle (0.2% tween 80 in saline), 5S-pioglitazone (as prepared in Example 6) (3 μg/kg), 5S-pioglitazone (as prepared in Example 6) (1 μg/kg), 5R-pioglitazone (as prepared in Example 4) (3 μg/kg), 5R-pioglitazone (as prepared in Example 4) (1 μg/kg), or racemic pioglitazone (3 μg/kg). The control group of mice received vehicle 1 hr prior to being exposed to air daily for a maximum of 50 minutes per day. BAL was performed 24 h following the final TS exposure. All compounds were dosed as the HCl salt with doses corrected as base.
In a second experiment, mice were dosed intra-nasally with vehicle (0.2% tween 80 in saline), 5S-Rosiglitazone (as prepared in Example 9) (3 μg/kg), 5S-Rosiglitazone (as prepared in Example 9) (10 μg/kg), 5R-Rosiglitazone (as prepared in Example 8) (3 μg/kg), 5R-Rosiglitazone (as prepared in Example 8) (10 μg/kg), or racemic Rosiglitazone (10 μg/kg). The control group of mice received vehicle 1 hr prior to being exposed to air daily for a maximum of 50 minutes per day. BAL was performed 24 h following the final TS exposure. All compounds were dosed as the HCl salt with doses corrected as free base.

Data management and statistical analysis

All results are presented as individual data points for each animal and the mean value was calculated for each group. Since tests for normality were positive, the data were subjected to a one way analysis of variance test (ANOVA), followed by a Bonferroni correction for multiple comparisons in order to test for significance between treatment groups. A "p" value of < 0.05 was considered to be statistically significant. Percentage inhibitions were automatically calculated within the Excel spreadsheets for the cell data using the formula below:

$$\%\ \text{Inhibition} = 1 - \left( \frac{\textit{Treatment group result} - \textit{sham group result}}{\textit{TS vehicle group result} - \textit{sham group result}} \right) \times 100$$

Inhibition data for other parameters were calculated manually using the above formula.
As illustrated in Figure 1, there was a clear difference in activity between the two enantiomers of pioglitazone on total cell BAL numbers following exposure to TS. The 5R enantiomer (e.e. 97.8%) of Pioglitazone significantly inhibited the BAL cell influx induced by TS at both 1 and 3 μg/kg when administered by the intranasal route. In contrast, the 5S-enantiomer (e.e. 97.5%) failed to inhibit the BAL cell inflammation at either dose examined.
Following examination of the BAL cell cytospins, BAL neutrophil numbers were determined. In concert with the activity on total BAL cells, the 5R enantiomer of pioglitazone significantly inhibited BAL neutrophil numbers induced by TS exposure at both doses whereas the 5S enantiomer of pioglitazone was ineffective (Figure 2).
Racemic pioglitazone (which contains 50% 5R enantiomer of pioglitazone) at the 3 μg/kg dose also significantly inhibited total and neutrophil BAL cells induced by TS.
As illustrated in Figure 3, there was a clear difference in activity between the two enantiomers of Rosiglitazone on total cell BAL numbers following exposure to TS. The 5R enantiomer (e.e. 85.7%) of Rosiglitazone significantly

inhibited the BAL cell influx induced by TS at both 3 and 10 μg/kg when administered by the intranasal route. In contrast, the 5S-enantiomer (e.e. 92.7%) failed to inhibit the BAL cell inflammation at either dose examined.

Following examination of the BAL cell cytospins, BAL neutrophil numbers were determined. In concert with the activity on total BAL cells, the 5R enantiomer of Rosiglitazone significantly inhibited BAL neutrophil numbers induced by TS exposure at both doses whereas the 5S enantiomer of Rosiglitazone was ineffective (Figure 2).

Racemic rosiglitazone (which contains 50% 5R enantiomer of Rosiglitazone) at the 10 μg/kg dose also significantly inhibited total and neutrophil BAL cells induced by TS.

Taken together, the results of the two studies identify the 5R enantiomer of both Pioglitazone and Rosiglitazone as possessing the anti-inflammatory activity required for inhibition of BAL cell influx whilst the 5S enantiomer does not.

Whilst the e.e. of the preparation of the 5R enantiomer of Rosiglitazone was lower than optimal (i.e., 85.7% rather than> 90%) differential activity of the two enantiomers was still observed. This suggests that an optimal preparation of 5R enantiomer of Rosiglitazone would show similar or even improved activity compared with the data presented herein. Therefore the data presented herein is indicative of what would be achieved with a preparation that contains by weight at least 95% 5R enantiomer of Rosiglitazone.

**Claims**

1. A glitazone for use in the treatment of inflammatory respiratory disease by pulmonary administration by inhalation, wherein the glitazone inhaled consists of at least 95% by weight of the 5R configuration and less than 5% by weight of the 5S configuration, and wherein the said glitazone is pioglitazone or rosiglitazone.

2. A glitazone for use as claimed in claim 1, wherein the said inflammatory respiratory disease is selected from mild asthma, moderate asthma, severe asthma, steroid resistant asthma, bronchitis, chronic obstructive pulmonary disease (COPD), cystic fibrosis, pulmonary edema, pulmonary embolism, pneumonia, pulmonary sarcoidosis, silicosis, pulmonary fibrosis, respiratory failure, acute respiratory distress syndrome, emphysema, chronic bronchitis, tuberculosis, and lung cancer.

3. A glitazone for use as claimed in claim 1, wherein the said inflammatory respiratory disease is chronic obstructive pulmonary disease

4. The use of a glitazone in the preparation of a medicament for the treatment of inflammatory respiratory disease by pulmonary administration by inhalation wherein the glitazone content of the medicament consists of at least 95% by weight of the 5R configuration and less than 5% by weight of the 5S configuration, and wherein the said glitazone is pioglitazone or rosiglitazone.

5. The use as claimed in claim 4, wherein the said inflammatory respiratory disease is selected from mild asthma, moderate asthma, severe asthma, steroid resistant asthma, bronchitis, chronic obstructive pulmonary disease (COPD), cystic fibrosis, pulmonary edema, pulmonary embolism, pneumonia, pulmonary sarcoidosis, silicosis, pulmonary fibrosis, respiratory failure, acute respiratory distress syndrome, emphysema, chronic bronchitis, tuberculosis, and lung cancer.

6. The use as claimed in claim 4, wherein the said inflammatory respiratory disease is chronic obstructive pulmonary disease

7. A pharmaceutical composition adapted for pulmonary administration by inhalation, which composition comprises a glitazone and one or more pharmaceutically acceptable carriers and/or excipients, and wherein the glitazone content of the composition consists of at least 95% by weight of the 5R configuration and less than 5% by weight of the 5S configuration, and wherein the said glitazone is pioglitazone or rosiglitazone.

8. A pharmaceutical composition as claimed in claim 7 which additionally comprises one or more other therapeutic agents selected from anti-inflammatory agents, bronchodilators, mucolytic agents, antitussive agents, leukotriene inhibitors, and antibiotics.

9. A kit for treatment of respiratory disorders in a subject, the kit comprising one dosage form comprising a composition as claimed in claim 7 and a second dosage form comprising another therapeutic agent selected from anti-inflammatory agents, bronchodilators, mucolytic agents, antitussive agents, leukotriene inhibitors, and antibiotics.

## Patentansprüche

1. Glitazon zur Verwendung bei der Behandlung von entzündlicher Atemwegserkrankung durch pulmonale Verabreichung durch Inhalation, wobei das eingeatmete Glitazon zu mindestens 95 Gew.-% aus der 5R-Konfiguration und zu weniger als 5 Gew.-% aus der 5S-Konfiguration besteht und wobei es sich bei dem Glitazon um Pioglitazon oder Rosiglitazon handelt.

2. Glitazon zur Verwendung nach Anspruch 1, wobei die entzündliche Atemwegserkrankung aus der Reihe mildes Asthma, mittelstarkes Asthma, starkes Asthma, steroidresistentes Asthma, Bronchitis, chronisch-obstruktive Lungenerkrankung (COPD), Mukoviszidose, Lungenödem, Lungenembolie, Lungenentzündung, Lungensarcoidose, Silikose, Lungenfibrose, akute respiratorische Insuffizienz, Atemnotsyndrom des Neugeborenen, Emphysem, chronische Bronchitis, Tuberkulose und Lungenkrebs ausgewählt ist.

3. Glitazon zur Verwendung nach Anspruch 1, wobei es sich bei der entzündlichen Atemwegserkrankung um die chronisch-obstruktive Lungenerkrankung handelt.

4. Verwendung eines Glitazons bei der Herstellung eines Arzneimittels für die Behandlung von entzündlicher Atemwegserkrankung durch pulmonale Verabreichung durch Inhalation, wobei der Glitazongehalt des Arzneimittels zu mindestens 95 Gew.-% aus der 5R-Konfiguration und zu weniger als 5 Gew.-% aus der 5S-Konfiguration besteht und wobei es sich bei dem Glitazon um Pioglitazon oder Rosiglitazon handelt.

5. Verwendung nach Anspruch 4, wobei die entzündliche Atemwegserkrankung aus der Reihe mildes Asthma, mittelstarkes Asthma, starkes Asthma, steroidresistentes Asthma, Bronchitis, chronisch-obstruktive Lungenerkrankung (COPD), Mukoviszidose, Lungenödem, Lungenembolie, Lungenentzündung, Lungensarcoidose, Silikose, Lungenfibrose, akute respiratorische Insuffizienz, Atemnotsyndrom des Neugeborenen, Emphysem, chronische Bronchitis, Tuberkulose und Lungenkrebs ausgewählt ist.

6. Verwendung nach Anspruch 4, wobei es sich bei der entzündlichen Atemwegserkrankung um die chronisch-obstruktive Lungenerkrankung handelt.

7. Pharmazeutische Zusammensetzung, die an die pulmonale Verabreichung durch Inhalation angepasst ist, wobei die Zusammensetzung ein Glitazon und einen oder mehrere pharmazeutisch unbedenkliche Träger und/oder Grundstoffe umfasst und wobei der Glitazongehalt der Zusammensetzung zu mindestens 95 Gew.-% aus der 5R-Konfiguration und zu weniger als 5 Gew.-% aus der 5S-Konfiguration besteht und wobei es sich bei dem Glitazon um Pioglitazon oder Rosiglitazon handelt.

8. Pharmazeutische Zusammensetzung nach Anspruch 7, die weiterhin ein oder mehrere zusätzliche therapeutische Mittel aus der Reihe entzündungshemmende Mittel, Bronchodilatatoren, schleimlösende Mittel, Antitussiva, Leukotrienhemmer und Antibiotika umfasst.

9. Kit für die Behandlung von Atemwegserkrankungen bei einem Patienten, wobei das Kit eine Arzneiform, umfassend eine Zusammensetzung nach Anspruch 7, und eine zweite Arzneiform, umfassend ein weiteres therapeutisches Mittel aus der Reihe entzündungshemmende Mittel, Bronchodilatatoren, schleimlösende Mittel, Antitussiva, Leukotrienhemmer und Antibiotika, umfasst.

## Revendications

1. Glitazone pour utilisation dans le traitement d'une maladie respiratoire inflammatoire par administration pulmonaire par inhalation, la glitazone inhalée étant constituée d'au moins 95 % en poids de la configuration 5R et de moins de 5 % en poids de la configuration 5S, et ladite glitazone étant la pioglitazone ou la rosiglitazone.

2. Glitazone pour utilisation selon la revendication 1, ladite maladie respiratoire inflammatoire étant choisie parmi l'asthme léger, l'asthme modéré, l'asthme grave, l'asthme résistant aux stéroïdes, la bronchite, la bronchopneumophatie chronique obstructive (BPCO), la mucoviscidose, l'oedème pulmonaire, l'embolie pulmonaire, la pneumonie, la sarcoïdose pulmonaire, la silicose, la fibrose pulmonaire, l'insuffisance respiratoire, le syndrome de détresse respiratoire aigu, l'emphysème, la bronchite chronique, la tuberculose, et le cancer du poumon.

**3.** Glitazone pour utilisation selon la revendication 1, ladite maladie respiratoire inflammatoire étant la bronchopneumophatie chronique obstructive.

**4.** Utilisation d'une glitazone dans la préparation d'un médicament pour le traitement d'une maladie respiratoire inflammatoire par administration pulmonaire par inhalation, la teneur en glitazone du médicament étant constituée d'au moins 95 % en poids de la configuration 5R et de moins de 5 % en poids de la configuration 5s, et ladite glitazone étant la pioglitazone ou la rosiglitazone.

**5.** Utilisation selon la revendication 4, dans laquelle ladite maladie respiratoire inflammatoire est choisie parmi l'asthme léger, l'asthme modéré, l'asthme grave, l'asthme résistant aux stéroïdes, la bronchite, la bronchopneumophatie chronique obstructive (BPCO), la mucoviscidose, l'oedème pulmonaire, l'embolie pulmonaire, la pneumonie, la sarcoïdose pulmonaire, la silicose, la fibrose pulmonaire, l'insuffisance respiratoire, le syndrome de détresse respiratoire aigu, l'emphysème, la bronchite chronique, la tuberculose, et le cancer du poumon.

**6.** Utilisation selon la revendication 4, dans laquelle ladite maladie respiratoire inflammatoire est la bronchopneumophatie chronique obstructive.

**7.** Composition pharmaceutique adaptée pour administration pulmonaire par inhalation, ladite composition comprenant une glitazone et un ou plusieurs véhicules et/ou excipients pharmaceutiquement acceptables, et la teneur en glitazone de la composition étant constituée d'au moins 95 % en poids de la configuration 5R et de moins de 5 % en poids de la configuration 5S, et ladite glitazone étant la pioglitazone ou la rosiglitazone.

**8.** Composition pharmaceutique selon la revendication 7 qui comprend en outre un ou plusieurs agents thérapeutiques choisis parmi des agents anti-inflammatoires, des bronchodilatateurs, des agents mucolytiques, des agents antitussifs, des inhibiteurs de leucotriène, et des antibiotiques.

**9.** Trousse pour le traitement de maladies respiratoires chez un sujet, la trousse comprenant une forme pharmaceutique comprenant une composition selon la revendication 7 et une deuxième forme pharmaceutique comprenant un autre agent thérapeutique choisi parmi des agents anti-inflammatoires, des bronchodilatateurs, des agents mucolytiques, des agents antitussifs, des inhibiteurs de leucotriène, et des antibiotiques.

Figure 1.

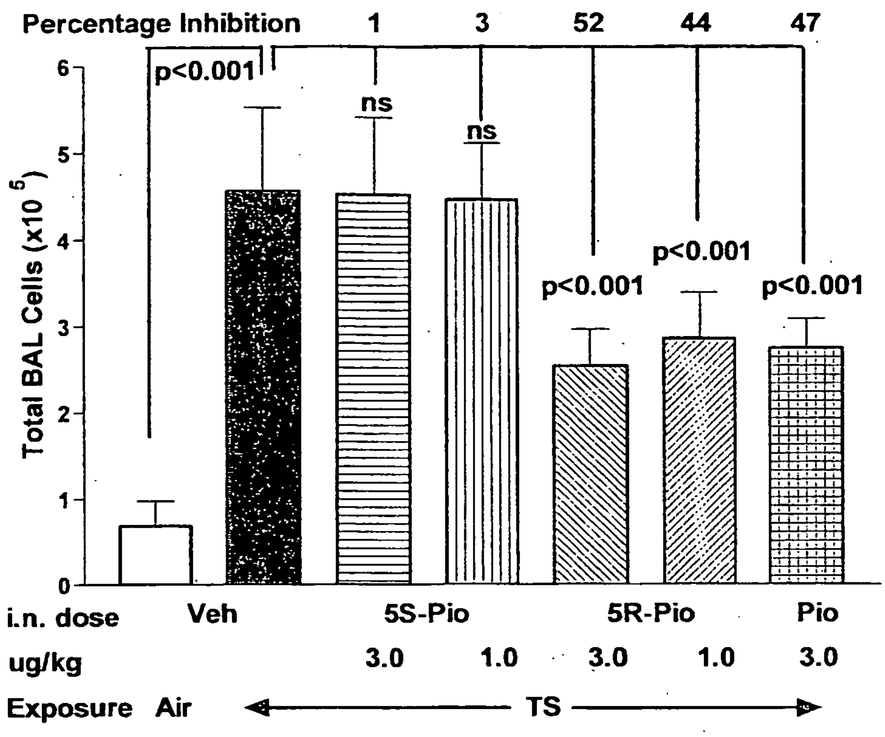


Figure 2.

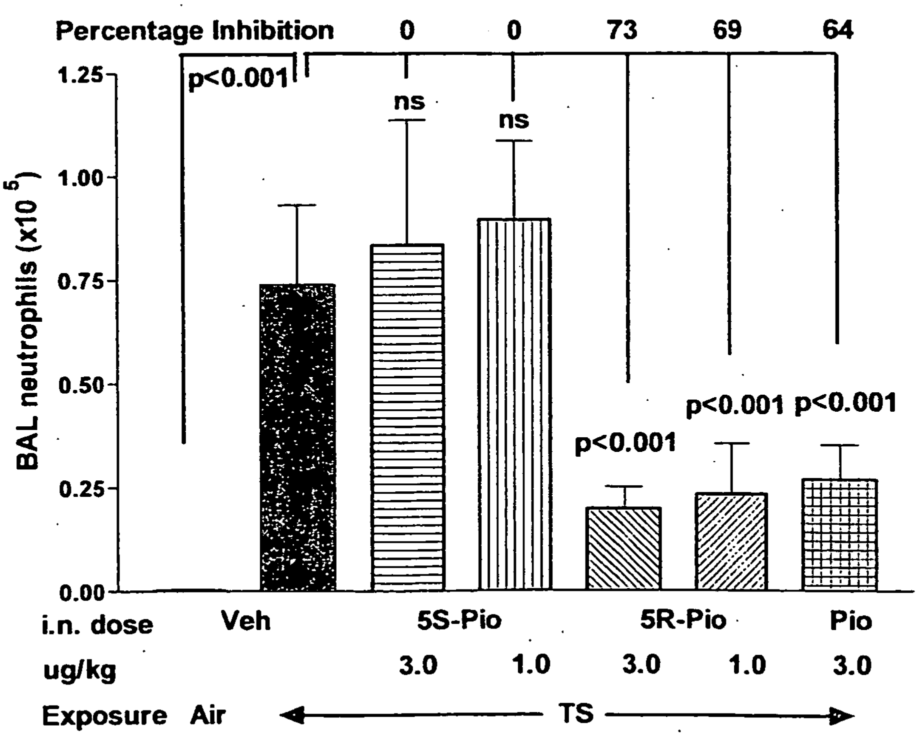

Figure 3.

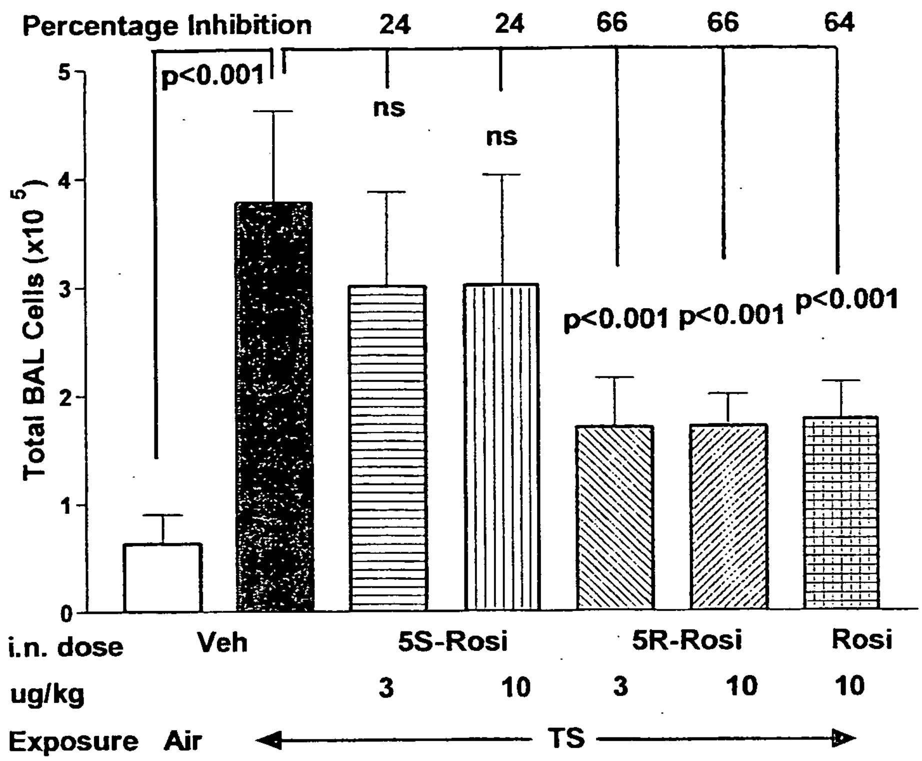


Figure 4.

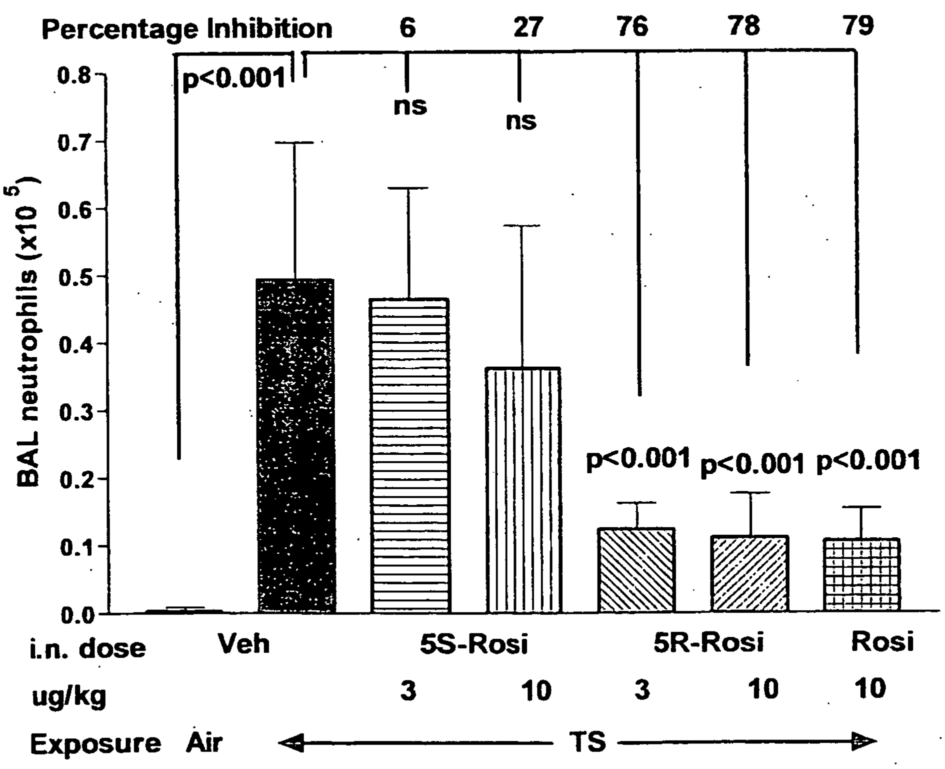

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0062766 A
- WO 0053601 A
- WO 0213812 A
- WO 2007100027 A
- WO 0212265 A
- WO 0212266 A
- WO 02100879 A
- WO 03062259 A
- WO 03048181 A
- WO 03042229 A
- WO 00032585 A
- WO 000210143 A
- WO 2005034939 A
- WO 2005003098 A
- WO 2005035518 A
- WO 2005035502 A
- EP 1440966 A
- JP 05025045 B
- WO 9318007 A
- WO 9964035 A
- US 20020055651 A
- US 20050133417 A
- US 20055159448 A
- WO 00075114 A
- WO 0142193 A
- WO 0183462 A
- WO 0266422 A
- WO 0270490 A
- WO 0276933 A
- WO 0324439 A
- WO 03142160 A
- WO 0342164 A
- WO 0372539 A
- WO 0391204 A
- WO 0399764 A
- WO 0416578 A
- WO 04016601 A
- WO 0422547 A
- WO 043292 A
- WO 0433412 A
- WO 0437768 A
- WO 0437773 A
- WO 0437807 A
- WO 0439762 A
- WO 0439766 A
- WO 0445618 A
- WO 0446083 A
- WO 0471388 A
- WO 0480964 A
- EP 1460064 A
- WO 04087142 A
- WO 0489892 A
- EP 01477167 A
- US 20040242622 A
- US 20040229904 A
- WO 04108675 A
- WO 04108676 A
- WO 05033121 A
- WO 05040103 A
- WO 05044787 A
- WO 04071388 A
- WO 05058299 A
- WO 05058867 A
- WO 05065650 A
- WO 05066140 A
- WO 05070908 A
- WO 05092840 A
- WO 05092841 A
- WO 05092860 A
- WO 05092887 A
- WO 05092861 A
- WO 05090288 A
- WO 05092087 A
- WO 05080324 A
- WO 05080313 A
- US 20050182091 A
- US 20050171147 A
- WO 05092870 A
- WO 05077361 A
- DE 10258695
- WO 05111002 A
- WO 05111005 A
- WO 05110990 A
- US 20050272769 A
- WO 05110359 A
- WO 05121065 A
- US 20060019991 A
- WO 06016245 A
- WO 06014704 A
- WO 06031556 A
- WO 06032627 A
- US 20060106075 A
- US 20060106213 A
- WO 06051373 A
- WO 06056471 A
- WO 08096112 A
- WO 08104790 A
- WO 08096119 A

- EP 0505321 A


**Non-patent literature cited in the description**


- **CAMPBELL IW.** *Curr Mol Med.,* May 2005, vol. 5 (3), 349-63
- **HAFFNER et al.** *Circulation,* 06 August 2002, vol. 106 (6), 679-84
- **MARX et al.** *Arterioscler. Thromb. Vasc. Biol.,* 01 February 2003, vol. 23 (2), 283-8
- **CUZZOCREA et al.** *Eur. J. Pharmacol.,* 01 January 2004, vol. 483 (1), 79-93
- **DESREUMANUX et al.** *J. Exp. Med.,* 02 April 2001, vol. 193 (7), 827-38
- **SANCHEZ-HIDALGO et al.** *Biochem. Pharmacol.,* 15 June 2005, vol. 69 (12), 1733-44
- **FEINSTEIN et al.** *Ann. Neurol.,* June 2002, vol. 51 (6), 694-702
- **CUZZOCREA et al.** *Arthritis Rheum.,* December 2003, vol. 48 (12), 3544-56
- **SHIOJIRI et al.** *Eur. J. Pharmacol.,* 19 July 2002, vol. 448 (2-3), 231-8
- **LEE et al.** *FASEB J.,* June 2005, vol. 19 (8), 1033-5
- **BIRRELL et al.** *Eur. Respir. J.,* July 2004, vol. 24 (1), 18-23
- **REDDY et al.** *Am. J. Physiol. Lung Cell. Mol. Physiol.,* March 2004, vol. 286 (3), L613-9
- **HETZEL et al.** *Thorax.,* September 2003, vol. 58 (9), 778-83
- **WARD.** *Br. J. Pharmacol.,* February 2004, vol. 141 (3), 517-25
- **MILAM et al.** *Am. J. Physiol. Lung Cell. Mol. Physiol,* 2008, vol. 294 (5), L891-901
- **CROSSNO et al.** *Am. J. Physiol. Lung Cell. Mol. Physiol,* 2007, vol. 292 (4), L885-897
- **GUAN et al.** *Nat. Med.,* 2005, vol. 11 (8), 861-6
- **ZHANG.** *Pro.c Natl. Acad. Sci. USA,* 2005, vol. 28 (26), 9406-11
- **CURKENDALL et al.** *Ann Epidemiol,* 2006, vol. 16, 63-70
- **PADELETTI M et al.** *Int J Cardiol.,* 2008, vol. 125 (2), 209-15
- **PARKS et al.** *Bioorg. Med. Chem. Lett.,* 1998, vol. 8 (24), 3657-8
- *J.Clin. Pharmacol.,* 2007, vol. 47, 323-33
- *Rapid Commun. Mass Spectrom.,* 2005, vol. 19, 1125-9
- *J. Chromatography,* 2006, vol. 835, 40-46
- *Biopharmaceutics and Drug Disposition,* 1997, vol. 18 (4), 305-24
- *Chem. Pharm. Bull,* 1984, vol. 32 (11), 4460-65
- *T. J. Med. Chem.,* 1991, vol. 34, 319-25
- **E. FOGASSY.** *Tetrahedron: asymmetry,* 2008, vol. 19, 519-536
- **S.H.WILEN.** Topics in Stereochemistry. Wiley-Interscience, 1972, vol. 6, 107
- Optical resolution Procedures of Chiral Compounds. Resolution Information Center, 1978, vol. 1-3
- **J.JAQUES ; S.H.WILEN ; A. COLLETT.** Enantiomers Racemates and Resolutions. Wiley-Interscience, 1991
- **R.A. SCHELDON.** Chirotechnology. Marcel Dekker, 1993
- Optical Resolutions via diastereomeric salt formation. CRC Press, 2002
- **K. AKIBA.** *Bull. Chem. Soc. Jpn.,* 1974, vol. 47 (4), 935-937
- **R.A. BARTSCH.** *JACS,* 2001, vol. 123 (31), 7479
- **T. SOHDA et al.** *Chem, Pharm. Bull.,* 1984, vol. 32 (11), 4460-5
- **J.R. FALCK et al.** *Bioorg.Med.Chem.Lett.,* 2008, vol. 18, 1768-1771
- *J. Chem. Soc. Perkin Trans. I,* 1994, 3319-3324
- *JACS,* 2004, vol. 126, 10216-7
- **EYLES JE ; WILLIAMSON ED ; ALPAR HO.** *Int J Pharm,* 1999, vol. 189 (1), 75-9